(19) **Europäisches Patentamt / European Patent Office / Office européen des brevets**

(11) **EP 1 244 704 B1**

(12) **EUROPEAN PATENT SPECIFICATION**

(45) Date of publication and mention
of the grant of the patent:
**13.08.2008 Bulletin 2008/33**

(51) Int Cl.:
**C07K 14/705** (2006.01)

(21) Application number: **00985581.8**

(22) Date of filing: **15.12.2000**

(86) International application number:
**PCT/GB2000/004828**

(87) International publication number:
**WO 2001/044296 (21.06.2001 Gazette 2001/25)**

(54) **METHODS OF INHIBITING THE BINDING OF A CD8+ T CELL TO A CLASS I MHC EMPLOYING A MODIFIED BETA 2 MICROGLOBULIN**

METHODEN UM DIE BINDUNG EINER CD8+ ZELLE AN EINEN CLASS I MHC ZU INHIBIEREN, UNTER VERWENDUNG EINES MODIFIZIERTEN BETA 2 MICROGLOBULINS

PROCEDES D'INHIBITION DE LA LIAISON D'UN LYMPHOCYTE CD8+ T AVEC UN MHC DE CLASSE I, METTANT EN OEUVRE UNE MICROGLOBULINE BETA 2 MODIFIEE

(84) Designated Contracting States:
**AT BE CH CY DE DK ES FI FR GB GR IE IT LI LU MC NL PT SE TR**

(30) Priority: **17.12.1999 GB 9929993**

(43) Date of publication of application:
**02.10.2002 Bulletin 2002/40**

(73) Proprietor: **Avidex Ltd**
**Abingdon,**
**Oxon OX14 4RX (GB)**

(72) Inventor: **JAKOBSEN, Bent Karsten**
**Abingdon,**
**Oxfordshire OX14 4RX (GB)**

(74) Representative: **Lee, Nicholas John et al**
**Kilburn & Strode**
**20 Red Lion Street**
**London WC1R 4PJ (GB)**

(56) References cited:
**WO-A-99/21576        WO-A-99/64597**
**US-A- 5 051 371        US-A- 5 674 681**

- **VAN EYNDHOVEN WINIFRED G ET AL: "Changes in rheumatoid factor and monoclonal IgG antibody specificity after site-specific mutations in antigenic region of beta-2-microglobulin." CLINICAL IMMUNOLOGY AND IMMUNOPATHOLOGY, vol. 72, no. 3, 1994, pages 362-372, XP000995144 ISSN: 0090-1229**

- **WILLIAMS RALPH C JR ET AL: "Antigenic epitopes on beta-2-microglobulin reacting with monoclonal antibodies." JOURNAL OF LABORATORY AND CLINICAL MEDICINE, vol. 121, no. 6, 1993, pages 805-820, XP000995194 ISSN: 0022-2143**

- **FUJIURA YASUYOSHI ET AL: "Development of CD8-alpha-alpha+ intestinal intraepithelial T cells in beta-2-microblobulin- and/or TAP1-deficient mice." JOURNAL OF IMMUNOLOGY, vol. 156, no. 8, 1996, pages 2710-2715, XP000995185 ISSN: 0022-1767**

- **GAO ET AL: "Assembly and crystallization of the complex between the human T cell coreceptor CD8alpha homodimer and HLA-A2" PROTEIN SCIENCE,GB,CAMBRIDGE UNIVERSITY PRESS, CAMBRIDGE, vol. 7, no. 5, 1 May 1998 (1998-05-01), pages 1245-1249, XP002093586 ISSN: 0961-8368**

- **GAO G F ET AL: "Crystal structure of the complex between human CD8alpha(alpha) and HLA-A2" NATURE,GB,MACMILLAN JOURNALS LTD. LONDON, vol. 387, no. 6633, 5 June 1997 (1997-06-05), pages 630-634, XP002093584 ISSN: 0028-0836**

- **SERREZE D V ET AL: "MAJOR HISTOCOMPATIBILITY COMPLEX CLASS I-DEFICIENT NOD-B2MNULL MICE ARE DIABETES AND INSULITIS RESISTANT" DIABETES,US, NEW YORK, NY, vol. 43, no. 3, 1 March 1994 (1994-03-01), pages 505-509, XP000603733 ISSN: 0012-1797**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- UGER ET AL: "Creating CTL targets with epitope-linked beta 2-microglobulin constructs" JOURNAL OF IMMUNOLOGY,US,THE WILLIAMS AND WILKINS CO. BALTIMORE, vol. 160, no. 4, 15 February 1998 (1998-02-15), pages 1598-1605, XP002115504 ISSN: 0022-1767

- PRINCE H E ET AL: "INTERRELATIONSHIPS BETWEEN SEROLOGIC MARKERS OF IMMUNE ACTIVATION AND T LYMPHOCYTE SUBSETS IN HIV INFECTION" JOURNAL OF ACQUIRED IMMUNE DEFICIENCY SYNDROMES, vol. 3, no. 5, 1990, pages 525-530, XP000995130 ISSN: 0894-9255

**Description**

The present invention relates to modified $\beta_2$-microglobulin ($\beta_2$m) proteins and in particular to their use in immunosuppressive therapy, in particular as inhibitors of cytotoxic T cell responses.

$\beta_2$-microglobulin forms a part of the Class I Major Histocompatibility Complex (MHC). MHC molecules are specialised protein complexes which present short protein fragments (peptide antigens) on the cell surface for recognition by the cellular arm of the adaptive immune system.

Class I MHC is a dimeric protein complex consisting of a variable heavy chain and a constant light chain, ($\beta_2$-microglobulin. Class I MHC presents peptides which are processed intracellularly, loaded into a binding cleft in the MHC, and transported to the cell surface where the complex is anchored in the membrane by the MHC heavy chain. Peptides are usually 8-11 amino acids in length, depending on the degree of arching introduced in the peptide when bound in the MHC. The binding cleft which is formed by the membrane distal $\alpha$1 and $\alpha$2 domains of the MHC heavy chain has "closed" ends, imposing quite tight restrictions on the length of peptide which can be bound.

($\beta_2$-microglobulin is a polypeptide which is found free in serum, which is non-covalently associated with MHC Class I molecules at the cell surface and which can exchange in the MHC complex with other free $\beta_2$m molecules (Bernabeu, et al. Nature 308: 642-5 (1984); Cook, et al. J Immunol 157: 2256-61 (1996); Horig, et al. Proc Natl Acad Sci U S A 94: 13826-31 (1997); Hyafil & Strominger, Proc Natl Acad Sci U S A 76: 5834-8 (1979); Luscher, et al. J Immunol 153: 5068-81 (1994); Parker, et al. J Immunol 149: 1896-904 (1992); Smith, et al. Proc Natl Acad Sci U S A 89: 7767-71 (1992)). Unlike MHC heavy chain polypeptides, $\beta_2$m is not anchored in the cell membrane. $\beta_2$m has the structure of an immunoglobulin C (constant) domain and also associates with a number of other class I - related structures, such as the products of the CD1 genes in man.

A wide spectrum of cells can present antigen, as MHC-peptide, and the cells which have that property are known as antigen presenting cells (APCs). The type of cell which presents a particular antigen depends upon how and where the antigen first encounters cells of the immune system. APCs include the interdigitating dendritic cells found in the T cell areas of the lymph nodes and spleen in large numbers; Langerhan's cells in the skin; follicular dendritic cells in B cell areas of the lymphoid tissue; monocytes, macrophages and other cells of the monocyte/macrophage lineage; B cells and T cells; and a variety of other cells such as endothelial cells and fibroblasts which are not classical APCs but can act in the manner of an APC.

APCs are recognised by a subgroup of lymphocytes which mature in the thymus (T cells) where they undergo a selection procedure ensuring that T cells which respond to self-peptides are eradicated (negative selection). In addition, T cells which do not have the ability to recognise the MHC variants which are presented (in man the HLA haplotypes) fail to mature (positive selection).

Recognition of specific MHC-peptide complexes by T cells is mediated by the T cell receptor (TCR) which consists of an $\alpha$- and a $\beta$ chain, both of which are anchored in the membrane. In a recombination process similar to that observed for antibody genes, the TCR $\alpha$- and $\beta$- genes rearrange from Variable, Joining, Diversity and Constant elements creating enormous diversity in the extracellular antigen binding domains ($10^{13}$ to $10^{15}$ different possibilities).

Antibody receptors and TCRs are the only two types of molecules which recognise antigens in a specific manner, and thus the TCR is the only receptor specific for particular peptide antigens presented in MHC, the alien peptide often being the only sign of an abnormality within a cell.

The vast majority of T cells restricted by Class I MHC-peptide complexes also require the engagement of the coreceptor CD8 for activation, while T cells restricted by Class II MHC require the engagement of CD4. The exact function of the coreceptors in T cell activation is not yet entirely clarified; neither are the critical mechanisms and parameters controlling activation. However, both CD8 and CD4 have cytoplasmic domains which are associated with the kinase p56$^{lck}$ which is involved in the very earliest tyrosine phosphorylation events which characterise T cell activation. CD8 is a dimeric receptor, expressed either in an $\alpha\alpha$ form or, more commonly, in an $\alpha\beta$ form. CD4 is a monomer. In the CD8 receptor only the $\alpha$-chain is associated with p56$^{lck}$. '

Recent determinations of the physical parameters controlling binding of TCR and CD8 to MHC, using soluble versions of the receptors, have shown that binding by TCR dominates the recognition event. TCR has significantly higher affinity for MHC than the coreceptors.

The individual interactions of the receptors with MHC are very short-lived at physiological temperature, i.e. 37°C. An approximate figure for the half life of a TCR-MHC/peptide interaction, measured with a human TCR specific for the influenza virus "matrix" peptide presented by HLA-A*0201 (HLA-A2), is 0.7 seconds. The half life of the CD8$\alpha\alpha$ interaction with the MHC/peptide complex is less than 0.01 seconds or at least 18 times faster (Willcox, et al. Immunity 10: 357-65 (1999); Wyer, et al. Immunity 10: 219-225 (1999)).

Class I MHC restricted cellular immune responses are mediated by cytotoxic T cells (CTLs). CTLs are activated through protein-protein interactions between T cell receptors (TCRs) and their coreceptors, CD8 molecules, on the T cell and MHC/peptide ligands on the target cell surface. TCRs form contacts both to the antigen peptide and to the heavy chain of the MHC complex (Ding, et al. Immunity 11: 45-56 (1999); Ding, et al. Immunity 8: 403-11 (1998); Garboczi, et al. Nature 384:

134-41 (1996); Garcia, et al. Science 274: 209-19 Issn: 0036-8075 (1996); and provide the antigen specificity of the T cell. CD8 binds to the heavy chain and the light chain ($\beta_2$-microglobulin) of the MHC complex, but not to the antigen peptide (Gao, et al. Nature 387: 630-4 (1997)). These protein binding events lead to activation of signal transduction in the T cell.

In contrast to antibody-antigen interactions, TCR-MHC/peptide interactions and, in particular, CD8-MHC interactions are characterised by low affinities and fast kinetics (Willcox, et al. Immunity 10: 357-65 (1999); Wyer, et al. Immunity 10: 219-225 (1999)). The interactions between T cells and antigen presenting cells are believed to be stabilised by multiple simultaneous receptor-ligand contacts, increasing the avidity of cell-cell interactions (Mescher, Immunol Rev 146, 177-210 (1995); Norment, el al. Nature 336: 79-81 (1988)).

Cytotoxic T cells are exquisitely sensitive to interference that disrupts the normal kinetics and/or affinities of the interactions required for activation. For example, altered peptide ligands (APLs) presented together with the normal antigen peptide on the MHC molecules of the antigen presenting cell can cause a failure to activate CTLs. Remarkably, this phenomenon can, in some cases, be observed even when the antagonist APL is presented at significantly lower molar ratios than the normal antigen (Bertoletti, et al. Nature 369: 407-10 Issn: 0028-0836 (1994); Klenerman, et al. Nature 369: 403-7 (1994); Purbhoo, et al. Proc. Natl. Acad. Sci. USA 95: 4527-4532 (1998)). Thus, CTLs are highly sensitive to variations in TCR-antigen contacts, even if the majority of these are within the 'normal' parameters required for activation.

Suppressors of the cellular arm of the immune system, such as suppressors of CD4 or CD8 T cells, are urgently needed for the treatment of auto-immune disorders, such as rheumatoid arthritis, lupus erthymatosus, psoriasis vulgaris, ankylosing spondylitis, Reiter's disease, post-salmonella arthritis, post-shigella arthritis, post-yersinia arthritis, post-gonococcal arthritis, uveitis, amylodosis, idiopathic hemachromatosis and myasthenia gravis, and the prevention of graft rejection and graft-versus-host disease. Antibodies directed against CD4 and CD8 have been tried (De Fazio, et al. Transplantation 61: 104-10 (1996)), but with limited success and antibodies in general are not well suited as drugs since they tend to induce secondary immune responses and are short-lived. Administration of steroids is another way of suppressing the immune system but their effect is extremely indirect and associated with severe side-effects.

Attempts have been made to modulate the binding of CD8 to MHC class I molecules. One previous attempt involved the *in vitro* use of peptides derived from HLA sequences thought to interact with CD8 (Clayberger, et al. J Immunol 153: 946-51 (1994)). Two CD8 derived peptides that were also tested were found to be incapable of suppressing the differentiation of human CTL precursors into active effector cells and unable to inhibit the subsequent action of these effector cells.

Another attempt to modulate CTL responses using free CD8 derived peptides (Choksi, et al. Nature Medicine 4: 309-314 (1998)) showed that one peptide in particular, "CSSHNKPC", could inhibit both the differentiation and effector stages of CTL response. However, a very high concentration of peptide (>100 $\mu$g/ml) was required to bring about this inhibition (>50%).

CTL inhibition has also been observed with soluble CD8$\alpha\alpha$ receptor (Sewell, et al. Nature Medicine 5: 399-404 (1999)). The inhibitory effect of the soluble CD8 molecule was more dramatic than that observed with an anti-CD8 monoclonal antibody.

In certain aspects, the present invention aims to prevent or inhibit CD8$^+$ T cell responses by preventing or inhibiting CD8 binding to these $\beta_2$-microglobulin subunit of the MHC/peptide complex.

According to a first aspect of the present invention, there is provided the use of a modified human $\beta_2$-microglobulin with a mutation at one or more of Serine$_{57}$, Lysine$_{58}$, Aspartate$_{59}$, Tryptophan$_{60}$ and Serine$_{61}$ whose binding to CD8 is inhibited in the manufacture of a medicament for inhibiting CD8$^+$ T cell response. Preferred features of the present invention are set out in the dependent claims herein.

In a second aspect, the invention provides a modified human $\beta_2$m, wherein: Lysine58 is replaced with Serine, Tyrosine, Tryptophan, Cysteine, Serine-Glutamate-Serine, or Glycine-Arginine-Glycine; or Aspartate$_{59}$ is replaced with Glycine-Glutamate-Glycine; or Lysine$_{58}$ is replaced with Arginine and Tryptophan$_{60}$ is replaced with Glycine.

Also disclosed herein is a method of inhibiting the binding of a CD8$^+$ T cell to a class I Major Histocompatibility Complex (MHC), the method comprising exposing the class I MHC to a modified $\beta_2$-microglobulin whose binding to CD8 is inhibited.

Also disclosed herein is a method for the treatment of an autoimmune disorder, graft versus host disease or graft rejection, comprising administering to a patient a modified $\beta_2$-microglobulin whose binding to CD8 is inhibited.

As used herein "inhibited", when used in the context of the binding of one entity to another, means that the binding is prevented altogether or reduced to such a level that the normal physiological results of binding cannot be observed or are not significant. Even low levels of binding inhibition can have severe physiological effects (for example, low levels of soluble CD8 receptor can cause CTL inhibition (Sewell, et al. Nature Medicine 5: 399-404 (1999)). It would be expected that, *in vitro*, the binding of soluble CD8 molecule to a soluble HLA complex refolded with a modified $\beta_2$-microglobulin may be inhibited by at least 20%, and more likely 50 to 100%. In a preferred embodiment, the present invention relates to a means of inhibiting CD8$^+$ T cell responses which is completely different to those proposed hitherto, in that CTLs are inhibited by preventing CD8 on the T cell surface from binding to MHC molecules on the antigen presenting cell by providing $\beta_2$-microglobulin which has been modified such that its binding to CD8 is impaired or inhibited. This may be achieved by providing $\beta_2$-microglobulin mutant proteins in which substitution, deletion and/or insertion mutations cause the binding of CD8 to be inhibited.

Substitution mutations are preferred as they create minimal disruption to the structure of $\beta_2$m. The mutated residues, for instance sterically and/or electrostatically, prevent or impair the binding of CD8 to MHC complexes involving the mutant $\beta_2$-microglobulin proteins. The observation that the inhibitory effect of soluble CD8 molecule is more dramatic than that observed with an anti-CDS monoclonal antibody (Sewell, et al. Nature Medicine 5: 399-404 (1999)) indicates that CTL activity is more susceptible to inhibition by interference with CD8 binding at the target cell surface than by interference at the T cell surface.

Because CD8 forms molecular contacts to both the MHC heavy chain and the light chain, $\beta_2$-Microglobulin, CD8 binding could, in principle, be inhibited by blocking the binding to either. However, the heavy chains of MHC complexes are membrane-bound and thus not well suited for the development of inhibitor proteins. $\beta_2$-microglobulin, on the other hand, is a soluble molecule that can be expressed as a recombinant protein (Gao, et al. Prot. Sci. 7: 1245-49 (1998); Garboczi, et al. Proc Natl Acad Sci U S A 89: 3429-33 Issn: 0027-8424 (1992); Parker & Wiley, Gene 83: 117-24 (1989)).

It is preferred if the modified $\beta_2$-microglobulin is derived from human $\beta_2$-microglobulin. The sequence for this protein is known (Swissprot P01884 EMBL/Genbank M17986). However, the modified $\beta_2$-microglobulin may be derived from any species, e.g. dog, cat, rat or mouse. The sequences for rat and mouse $\beta_2$-microglobulin are known (Rat: Swissprot P07151, EMBL/Genbank Y00441, Mouse: Swissprot P01887 EMBL/Genbank X01838). For those $\beta_2$-microglobulins which have not been crystallised and hence do not have data available as to which residues thereof are involved in binding CD8, the skilled person can identify likely candidate residues for mutation using homology studies.

Mutated versions of the $\beta_2$m protein are known. However, none of these have been suggested for inhibiting class I MHC complex contacts to CD8. The most comprehensive set of mutations was investigated by Fukuzawa et al (Fukazawa, et al. J Immunol 153: 3543-50 (1994)) who generated 18 mutant $\beta_2$m proteins. The aim of this study was to alter the conformation of HLA-B27 in order to change the peptide presentation of the HLA complex. HLA-B27 is strongly associated with ankylosing spondylitis (Ivanyi, Curr Opin Rheumatol 4: 484-93 (1992); Reveille, Am J Med Sci 316: 239-49 (1998)) and is also associated with an increased frequency of certain autoimmune diseases (Altman, et al. Ann Allergy 72: 307-16 (1994); Reveille, Am J Med Sci 316: 239-49 (1998)). The aim of Fukuzawa et al was to investigate whether T cell recognition of HLA-B27 could be modulated by $\beta_2$m mutant proteins, the idea being that the structure of the peptide binding groove of the HLA heavy chain may be affected by amino acid substitutions in $\beta_2$m (Fukazawa, et al. J Immunol 153: 3543-50 (1994)). The residues in the human $\beta_2$m that were mutated were positions 2, 4, 6, 10, 31, 32, 33, 51, 52 , 53, 54, 55, 56, 59, 60, 63, 64, and 65. Some of these were only mutated in combination with other residues. Some mutations affected binding of a monoclonal antibody, Ye-2, to the HLA-B27 complex. The majority of the mutated proteins had reduced ability to exchange with $\beta_2$m on the cell surface, probably because the mutations affected residues involved in $\beta_2$m-heavy chain contacts (Fukazawa, et al. J Immunol 153: 3543-50 (1994)). The paper concludes that "It is unlikely that any of the mutants can be effective therapeutically in altering HLA-B27 function. Those mutants that are effective also exchange poorly into the complex." The authors only consider the possibility of modulating CTL activity through the influence $\beta_2$m mutations may have on peptide antigen presentation. The manuscript does not mention that mutations in $\beta_2$m might be used to modulate CTL activity by altering interactions with CD8. Furthermore, mutations at position 58 in $\beta_2$m are unlikely to have an adverse effect on the ability of the mutated $\beta_2$m polypeptide to exchange into HLA complexes as was observed with the majority of the mutant proteins analysed by Fukuzawa *et al.*

Another study has analysed the effects of single-residue mutations in the epitope of $\beta_2$m recognised by a panel of polyclonal antisera and by monoclonal antibodies (van Eyndhoven, et al. Clin Immunol Immunopathol 72: 362-72 (1994)). Mutations were concentrated in the region constituted by residues 57-63 of $\beta_2$m. The manuscript concludes 'that single residues within a small seven amino acid, solvent accessible antigenic stretch of primary beta $_2$m sequence vary considerably in their contribution to the reactive antigenic structures for IgM RF as well as monoclonal IgG antibodies.' The manuscript does not address the effect of the mutations on $\beta_2$m exchange, on HLA structure or on CD8 recognition.

A more recent study used murineP2M mutants in exchange assays, followed by antibody recognition (Schultz, et al. Immunogenetics 48: 273-82 (1998)). This study did not address the issues of CTL reactivity or CD8 inhibition. Finally, a mutation of Tyrosine$_{67}$ to Cysteine in human $\beta_2$m has been described (Walter, et al. J Immunol Methods 214: 41-50 (1998)). The introduced Cysteine residue was used for adding a biotin to the SH group, allowing soluble HLA complexes to be tetramerised through the linkage of biotin to avidin (Walter, et al. J Immunol Methods 214: 41-50 (1998)). Trymbulak et al, Immunogenetics 46: 418-426 (1997) also describes mutations to $\beta_2$m.

A modified human $\beta_2$m may have Lysine$_{58}$ replaced with one or more of Histidine, Tryptophan, Tyrosine, Phenylalanine, Glutamate, Aspartate, Glycine, Alanine, Valine, Leucine, Isoleucine, Methionine, Serine, Threonine, Asparagine, Glutamine, Proline and Cysteine. It is preferred if Lysine$_{58}$ is replaced with Arginine, Serine, Tyrosine, Tryptophan, Cysteine, Serine-Glutamate-Serine, or Glycine-Arginine-Glycine, and especially preferred if replaced with Glutamate.

A modified human $\beta_2$m may have Serine$_{57}$ replaced with one or more of Arginine, Histidine, Tryptophan, Tyrosine, Phenylalanine, Glutamate, Aspartate, Glycine, Alanine, Valine, Leucine, Isoleucine, Methionine, Asparagine, Glutamine, Proline and Cysteine.

A modified human $\beta_2$m may have Aspartate$_{59}$ replaced with one or more of Arginine, Histidine, Tryptophan, Tyrosine, Phenylalanine, Glutamate, Glycine, Alanine, Valine, Leucine, Isoleucine, Methionine, Asparagine, Glutamine, Proline and Cysteine, provided that, when mutated to Alanine, Phenylalanine56 is not mutated to Tyrosine. It is preferred if Aspartate$_{59}$ is replaced with Glycine-Glutamate-Glycine.

A modified human $\beta_2$m may have Tryptophan$_{60}$ replaced with one or more of Arginine, Histidine, Tyrosine, Phenylalanine, Glutamate, Aspartate, Glycine, Alanine, Valine, Leucine, Isoleucine, Methionine, Serine, Threonine, Asparagine, Glutamine, Proline and Cysteine, provided that:

> when mutated to Arginine, (i) Leucine$_{54}$ is not mutated to Valine and/or Leucine$_{64}$ is not mutated to Arginine;
>
> when mutated to Cysteine, another residue is also mutated;
>
> when mutated to Leucine, Histidine$_{51}$ is not mutated to Tyrosine; and
>
> when mutated to Glycine, Aspartate$_{53}$ is not mutated to Tyrosine. It is preferred if Tryptophan$_{60}$ is replaced with Glycine.

A modified human $\beta_2$m may have Serine$_{61}$ replaced with one or more of Arginine, Histidine, Tryptophan, Tyrosine, Glutamate, Aspartate, Glycine, Alanine, Valine, Leucine, Isoleucine, Methionine, Threonine, Asparagine, Glutamine, Proline and Cysteine.

A modified human $\beta_2$m may have Lysine$_{58}$ replaced with Arginine and Tryptophan$_{60}$ is replaced with Glycine.

Unless the context dictates otherwise, reference to "human $\beta_2$m" means a protein comprising amino acid sequence 1-99 in Figure la, and reference to numbered amino acid residues in human $\beta_2$m is in accordance with the numbering of the amino acid residues in Figure la. A modified human $\beta_2$m may have a combination of one or more of the mutations described above. The specific mutations mentioned may be the only mutations. Alternatively, there may be other mutations.

A modified human $\beta_2$m of the present invention may be provided in substantially pure form. For example, it may be provided in a form which is substantially free of other proteins.

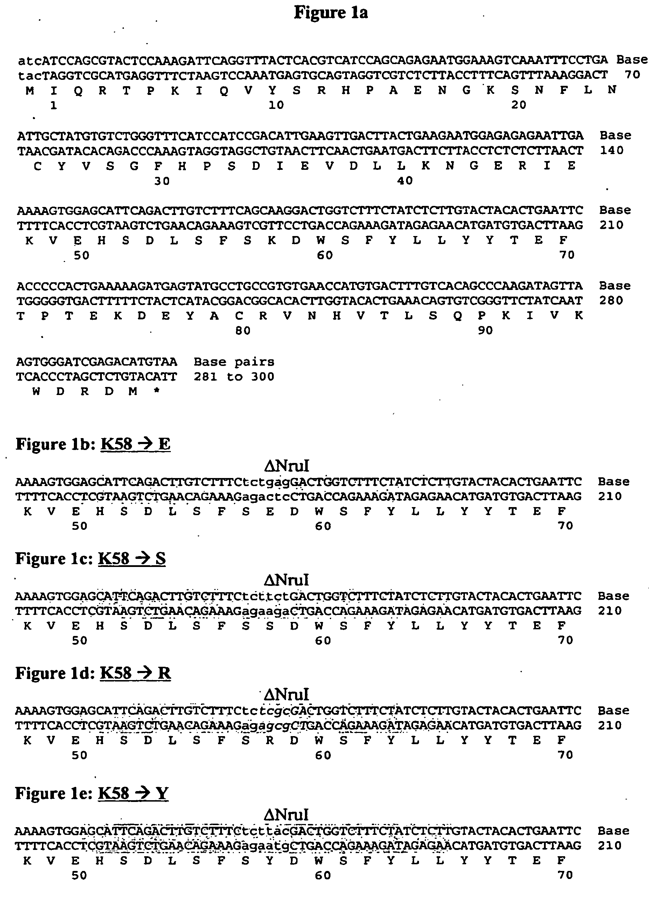

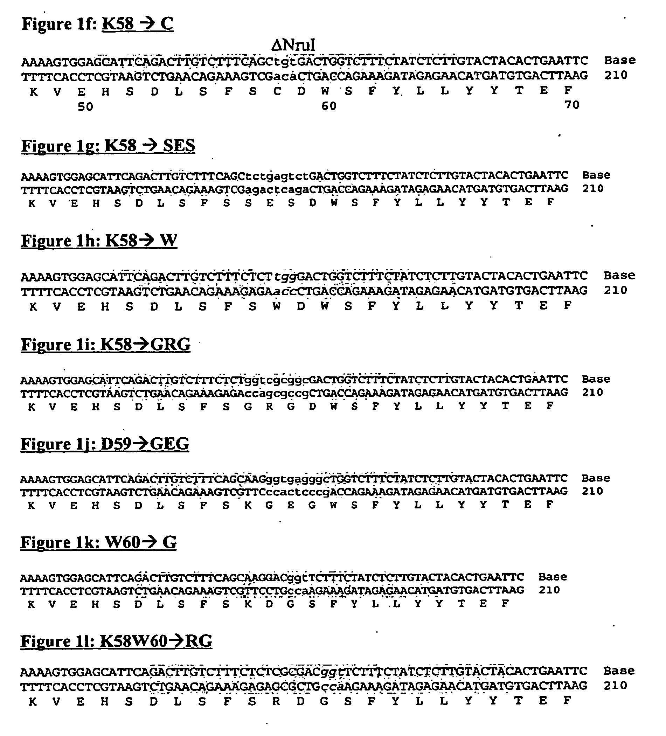

Also disclosed is any protein coded for by the nucleic acid sequences as shown in Figure la herein, when modified as shown in any one of Figures 1b-11.

The skilled person will appreciate that homologues or derivatives of the modified human $\beta_2$m proteins described above will also find use in the context of the present invention, i.e. in inhibiting the binding of a CD8$^+$ T cell to a class I Major Histocompatibility Complex (MHC). Thus, for instance proteins which include one or more additions, deletions, substitutions or the like are encompassed by the present invention. In addition, it may be possible to replace one amino acid with another of similar "type". For instance, replacing one hydrophobic amino acid with another. One can use a program such as the CLUSTAL program to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of analysis are contemplated in the present invention.

In the case of homologues and derivatives, the degree of identity with a protein as described herein is less important than that the homologue or derivative should retain the ability to inhibit the binding of a CD8$^+$ T cell to a class I Major Histocompatibility Complex. However, suitably, homologues or derivatives having at least 60% similarity (as discussed above) with the proteins or polypeptides described herein are provided. Preferably, homologues or derivatives having at least 70% similarity, more preferably at least 80% similarity are provided. Most preferably, homologues or derivatives having at least 90% or even 95% similarity are provided.

In an alternative approach, the homologues or derivatives could be fusion proteins, incorporating moieties which render purification easier, for example by effectively tagging the desired protein or polypeptide. It may be necessary to remove the "tag" or it may be the case that the fusion protein itself retains sufficient ability to inhibit the binding of aCD8$^+$ T cell to a class I Major Histocompatibility Complex to be useful.

The modified human $\beta_2$M proteins useful in the present invention can be provided alone, as a purified or isolated preparation. They may be provided as part of a mixture with one or more other proteins of the invention.

Also described are:

> (a) the use of a modified human $\beta_2$m protein described herein in inhibiting the binding of a CD8$^+$ T cell to a class I Major Histocompatibility Complex;
>
> (b) the use of a modified human $\beta_2$m protein de-

scribed herein in the production of a medicament for inhibiting CD8$^+$ T cell response; and

(c) a method of inhibiting the binding of a CD8$^+$ T cell to a class I Major Histocompatibility Complex (MHC), the method comprising exposing the class I MHC to a modified human $\beta_2$m protein described herein.

Gene cloning techniques may be used to provide a modified human $\beta_2$m protein of the invention in substantially pure form. These techniques are disclosed, for example, in J. Sambrook et al Molecular Cloning 2nd Edition, Cold Spring Harbor Laboratory Press (1989). Thus, a nucleic acid molecule may be provided comprising or consisting of a sequence which is:

(i) the DNA sequence set out in Figure la herein, when modified as shown in any one of Figures 1b-11, or its RNA equivalent;

(ii) a sequence which is complementary to the sequence of (i);

(iii) a sequence which codes for the same protein or polypeptide, as the sequence of (i) or (ii);

(iv) a sequence which is has substantial identity with any of those of (i), (ii) and (iii);

(v) a sequence which codes for a homologue, derivative or fragment of a protein as defined in Figure 1a, when modified as shown in any one of Figures 1b-11.

The nucleic acid molecules may include a plurality of such sequences, and/or fragments. The skilled person will appreciate that novel variants of those particular novel nucleic acid molecules which are exemplified herein can be provided. These may occur in nature, for example because of strain variation. For example, additions, substitutions and/or deletions are included. In addition, and particularly when utilising microbial expression systems, one may wish to engineer the nucleic acid sequence by making use of known preferred codon usage in the particular organism being used for expression. Thus, synthetic or non-naturally occurring variants are also included within the scope of the invention.

The term "RNA equivalent" when used above indicates that a given RNA molecule has a sequence which is complementary to that of a given DNA molecule (allowing for the fact that in RNA "U" replaces "T" in the genetic code).

When comparing nucleic acid sequences for the purposes of determining the degree of homology or identity, one can use programs such as BESTFIT and GAP (both from the Wisconsin Genetics Computer Group (GCG) software package). BESTFIT, for example, compares two sequences and produces an optimal alignment of the most similar segments. GAP enables sequences to be aligned along their whole length and finds the optimal alignment by inserting spaces in either sequence as appropriate. Suitably, in the context of the present invention compare when discussing identity of nucleic acid sequences, the comparison is made by alignment of the sequences along their whole length.

Preferably, sequences which have substantial identity have at least 50% sequence identity, desirably at least 75% sequence identity and more desirably at least 90 or at least 95% sequence identity with said sequences. In some cases, the sequence identity may be 99% or above.

Desirably, the term "substantial identity" indicates that said sequence has a greater degree of identity with any of the sequences described herein than with prior art nucleic acid sequences.

Substantially identical sequences may hybridise with the sequences of (i), (ii) and (iii) above under moderate or stringent hybridising conditions.

It should however be noted that, where a nucleic acid sequence codes for at least part of a novel gene product, the present invention includes within its scope all possible sequence coding for the gene product or for a novel part thereof.

The nucleic acid molecule may be in isolated or recombinant form. It may be incorporated into a vector and the vector may be incorporated into a host. Such vectors and suitable hosts form yet further aspects of the present invention.

As mentioned above, soluble $\beta_2$-microglobulin protein, added to class I antigen presenting cells, is known to exchange with the protein that is part of the MHC complexes on the cell surface (Bernabeu, et al. Nature 308: 642-5 (1984) ; Cook, et al. J Immunol 157: 2256-61 (1996); Horig, et al. Proc Natl Acad Sci U S A 94:13826-31 (1997); Hyafil & Strominger, Proc Natl Acad Sci U S A 76: 5834-8 (1979); Luscher, et al. J Immunol 153: 5068-81 (1994); Parker, et al. J Immunol 149: 1896-904 (1992); Smith, et al. Proc Natl Acad Sci U S A 89: 7767-71 (1992)). Experiments with a cell line have indicated that 10-25% of the exchange takes place within 10-15 minutes after addition of exogenous $\beta_2$m (Luscher, et al. J Immunol 153: 5068-81 (1994)). Importantly, $\beta_2$m exchange does not affect the stability of peptide binding, so antigen presentation is not lost or altered on the MHC molecules which undergo $\beta_2$m exchange (Cook, et al. J Immunol 157: 2256-61 (1996) ; Horig, et al. Proc Natl Acad Sci U S A 94: 13826-31 (1997) ; Parker, et al. J Immunol 149: 1896-904 (1992); Smith, et al. Proc Natl Acad Sci U S A 89: 7767-71 (1992)). Thus, recombinant $\beta_2$-microglobulin proteins, modified or mutated so as to prevent CD8 forming the normal binding complex with the MHC (heavy chain/light chain/peptide) molecule, can be added to antigen presenting cells to act as an immune inhibitor. The modified $\beta_2$m can be delivered to the antigen presenting cell surface either by exchange or by ex-

pression of a mutated $\beta_2$m gene in the cells, as is explained in more detail below.

Binding of the CD8$\alpha\alpha$ homodimer to the class I HLA complex is asymmetric, the two subunits of CD8 being involved in different contacts. Only one of the CD8 subunits is involved in binding $\beta_2$m, through its $\beta$ strand 'A' in the immunoglobulin structure. The contributions of the $\beta_2$m-CD8$\alpha$ contacts to binding have not been established. However, considering the low affinity of CDB$\alpha\alpha$ binding to HLA complex (Wyer, et al. Immunity 10: 219-225 (1999)), it is likely that disruption of any contacts will lead to a significant inhibition of coreceptor's contribution to signal transduction in T cells.

Two residues in human $\beta_2$-microglobulin are the preferred targets for mutation. These are Lysine$_{58}$ and Tryptophan$_{60}$, both of which are involved in contacts with CD8 (Gao, et al. Nature 387: 630-4 (1997)). Lysine$_{58}$ is the most preferred target. CD8 fits tightly into the binding 'cavity' formed by the heavy chain/light chain complex (Gao, et al. Nature 387: 630-4 (1997)). In the CD8$\alpha\alpha$-HLA-A2 crystal structure, Lysine$_{58}$ protrudes from the $\beta_2$m loop towards the cavity occupied by CD8 (Gao, et al. Nature 387: 630-4 (1997)) and forms contacts to the CD8 residues Arginine$_4$, Valine$_{24}$, Leucine$_{25}$, Leucine$_{26}$ and Aspartate$_{75}$. Therefore, amino acids with larger side chains, for instance arginine or tyrosine, at position 58 of $\beta_2$-microglobulin have severe effects on CD8 binding. Aspartic acid or glutamic acid residues may also be used for substituting Lysine$_{58}$, since these exercise electrostatic repulsion of CD8. Even fairly inconspicuous residues inserted in place of Lysine$_{58}$, for example serine, abrogate CD8 binding due to loss of specificity. In addition, CD8 binding may be inhibited by deleting Lysine$_{58}$ or inserting one or more resides which disrupt the cavity occupied by CD8.

It is known that mutations in the $\beta_2$m polypeptide can severely impair its ability to exchange into the HLA complex. One residue, mutation of which was found to impair the ability of the protein to exchange, was Tryptophan$_{60}$ (Fukazawa, et al. J Immunol 153: 3543-50 (1994)). However; Lysine$_{58}$ is located in a loop, not a strand, of $\beta_2$m and is not involved in contacts with the class I heavy chain. Therefore amino acid substitutions of Lysine$_{58}$ should not impair the ability of the protein to exchange into the HLA complex. Lysine$_{58}$ is therefore the prime candidate residue for substitutions designed to generate a soluble protein, that can exchange with $\beta_2$m on the cell surface and serve to inhibit CTL activation by impairing CD8 contacts to the MHC complex.

The preferred mutations of Lysine$_{58}$ and the rationale for how they might change CD8 recognition, and inhibit this, are as follows:

Lysine$_{58} \to$ Arginine (R). Arginine has a larger, and different, side chain than lysine, and therefore this substitution is likely to sterically hinder CD8 from slotting correctly into the cavity formed by the HLA complex.

Lysine$_{58} \to$ Glutamate (E). The side chain of glutamic acid is oppositely charged to lysine, and therefore this substitution is likely to electrostatically repel CD8 binding to HLA complexes.

Lysine$_{58} \to$ Serine (S). The side chain of serine lacks the positive charge of the lysine side chain. The lack of the electrostatic interactions to CD8 in which Lysine$_{58}$ is involved is probably sufficient to weaken CD8 binding to HLA complexes.

Lysine$_{58} \to$ Tyrosine (Y). Tyrosine has a substantially larger, and under normal conditions uncharged, side chain than lysine. This substitution is likely to sterically hinder CD8 binding to HLA complexes.

Lysine$_{58} \to$ Cysteine (C). Mutation to cysteine creates a 'free' SH group on the surface of $\beta_2$m being contacted by CD8. This may in itself inhibit CD8 contacts, but the residue could also be used to derivatise the $\beta_2$m polypeptide.

Figure 1a of the accompanying drawings shows the amino acid sequence of the processed human $\beta_2$m polypeptide and the DNA sequence encoding it, and Figures 1b-1 show the modifications that need to be made to this DNA sequence to introduce the mutations described above, as well as other mutations, and the amino acid sequences of the relevant regions of resulting $\beta_2$m proteins.

Tryptophan$_{60}$ is another preferred residue for mutation as it is involved in forming a contact to Arginine$_4$ in CD8$\alpha$ (Gao et al., Nature 387:630-4 (1997)). However, data published in Fukazawa, et al. J Immunol 153: 3543-50 (1994) suggests that mutation of this residue may interfere with the ability of the mutant protein to exchange. Thus, it is preferred that such mutant proteins are exposed to antigen presenting cells by means other than exchange, such as by expression of the mutant protein in the cell.

In addition to Lysine$_{58}$ and Tryptophan$_{60}$, because of the low affinity and transient nature of CD8-MHC interactions, functionally significant effects may be achieved by quite a large number of mutations (whether they be insertion, deletion or substitution mutations) in residues 57-61 of human $\beta_2$m (i.e. Serine$_{57}$, Aspartate$_{59}$ and Serine$_{61}$) either singly or in combination.

Substitution mutations of these residues can be divided into categories as follows:

• Mutation to Arginine, Histidine, Tryptophan, Tyrosine or Phenylalanine. These residues have large side chains and are therefore likely to cause steric obstruction of CD8 binding if introduced into the loop contacted by the coreceptor.

• Mutation to Glutamate or Aspartate. These residues have negatively charged side chains and are likely to cause electrostatic repulsion of CD8 binding, since Aspartate$_{75}$ of human CD8$\alpha$ is involved in contacts to $\beta_2$m (Gao, et al. Nature 387: 630-4 (1997)).

• Mutation to Glycine, Alanine, Valine, Leucine, Isoleucine, Methionine, Serine, Threonine, Asparagine or Glutamine. Some of these residues have small side chains, and all are uncharged at normal physiological pH. Mutations of one or several of residues 57-61 of human $\beta_2$m to any of this set of residues could cause loss of CD8 binding, either sterically or simply because the normal contacts between CD8 and $\beta_2$m are lost.

• Mutation to Proline. Proline induces structural restrictions in polypeptides and would therefore be predicted to alter the structure of the 57-61 loop of human $\beta_2$m, with expected loss of CD8 binding to the MHC complex. However, introduction of a Proline residue(s) may not be compatible with a functional $\beta_2$m structure, that is, folding of $\beta_2$m may be affected in such a way that the protein cannot form complexes with HLA heavy chains or have impaired ability to exchange into existing complexes on surface of cells.

• Mutation to Cysteine. Mutation to cysteine would create a 'free' SH group on the surface of $\beta_2$m being contacted by CD8. This may in itself inhibit CD8 contacts, but the residue could also be used to derivatise the $\beta_2$m polypeptide. For instance, a biotin group could be linked to $\beta_2$m, causing steric hindrance of CD8 binding if the mutated, derivatisedss2m polypeptide was exchanged into MHC complexes.

In addition to the mutations described above, $\beta_2$m may be mutated to increase its affinity for MHC so that its on-rate is greater and its off-rate is less than wild type $\beta_2$m, thereby resulting in greater occupancy of the mutantss2m in the MHC. Mutations of this type are described in Schultz, et al. Immunogenetics 48: 273-82 (1998).

Other possible mutations will be apparent to those skilled in the art.

Mutant $\beta_2$m proteins useful in the present invention can be expressed as soluble recombinant protein for extracellular addition, or expressed intracellularly by transfection of a DNA construct encoding the mutant $\beta_2$m protein. Transfection of DNA can be achieved both in vitro as well as in vivo, for example by using various type of recombinant viruses as vehicles for DNA transformation or by transfection techniques that use 'naked' DNA. For example, an organ to be transplanted may be incubated in a modified $\beta_2$m to make it more difficult for the immune system of host to recognise and reject it.

Preferably in gene therapy, the modified $\beta_2$m protein is administered such that it is expressed in the subject to be treated for example in the form of a recombinant DNA molecule comprising a polynucleotide encoding the modified $\beta_2$m protein operatively linked to a nucleic acid sequence which controls expression, such as in an expression vector. Such a vector will thus include appropriate transcriptional control signals including a promoter region capable of expressing the coding sequence, said promoter being operable in the subject to be treated. Thus for human gene therapy, the promoter, which term includes not only the sequence necessary to direct RNA polymerase to the transcriptional start site, but also, if appropriate, other operating or controlling sequences including enhancers, is preferably a human promoter sequence from a human gene, or from a gene which is typically expressed in humans, such as the promoter from human cytomegalovirus (CMV). Among known eukaryotic promoters suitable in this regard are the CMV immediate early promoter, the HSV thymidine kinase promoter, the early and late SV40 promoters, the promoters of retroviral LTRs, such as those of the Rous sarcoma virus ("RSV"), and metallothionein promoters, such as the mouse metallothionein-I promoter.

A polynucleotide sequence and transcriptional control sequence may be provided cloned into a replicable plasmid vector, based on commercially available plasmids, such as pBR322, or may be constructed from available plasmids by routine application of well known, published procedures.

The vector may also include transcriptional control signals, situated 3' to the mutant $\beta_2$m encoding sequence, and also polyadenylation signals, recognisable in the subject to be treated, such as, for example, the corresponding sequences from viruses such as, for human treatment, the SV40 virus. Other transcriptional controlling sequences are well known in the art and may be used.

The expression vectors may also include selectable markers, such as for antibiotic resistance, which enable the vectors to be propagated.

Expression vectors capable in situ of synthesising mutant $\beta_2$m may be introduced directly by physical methods. Examples of these include topical application of the 'naked' nucleic acid vector in an appropriate vehicle for example in solution in a pharmaceutically acceptable excipient such as phosphate buffered saline (PBS). Other physical methods of administering the DNA directly to the recipient include ultrasound, electrical stimulation, electroporation and microseeding.

Mutant $\beta_2$m encoding nucleic acid sequence for use in therapy may also be administered by means of delivery vectors. These include viral delivery vectors, such as adenovirus or retrovirus, delivery vectors known in the art. Other nonviral delivery vectors include lipid delivery vectors, including liposome delivery vehicles, known in the art.

A mutant $\beta_2$m encoding nucleic acid sequence may also be administered by means of transformed host cells. Such cells include cells harvested from the subject, into which the nucleic acid sequence is introduced by gene transfer methods known in the art, followed by growth of the transformed cells in culture and administration to the subject.

Expression constructs such as those described above may be used in a variety of ways in the therapy of the present invention. Thus, they may be directly administered to the subject, or they may be used to prepare

mutant $\beta_2$m itself which can then be administered as is discussed in more detail below. The invention also relates to host cells which are genetically engineered with constructs which comprise mutant $\beta_2$m encoding polynucleotide, and to the uses of these vectors and cells in the therapeutic methods of the invention. These constructs may be used *per se* in the therapeutic methods of the invention or they may be used to prepare a mutant $\beta_2$m polypeptide for use in the therapeutic methods of the invention described in greater detail below.

The vector may be, for example, a plasmid vector, a single or double-stranded phage vector, a single or double-stranded RNA or DNA viral vector, depending upon whether the vector is to be administered directly (i.e. for *in situ* synthesis), or is to be used for synthesis of mutant $\beta_2$m. Starting plasmids disclosed herein are either commercially available, publicly available, or can be constructed from available plasmids by routine application of well known, published procedures. Many plasmids and other cloning and expression vectors that can be used in accordance with the present invention are well known and readily available to those of skill in the art.

Generally, vectors for expressing a mutant $\beta_2$m polypeptide for use in the invention comprise cis-acting control regions effective for expression in a host operatively linked to the polynucleotide to be expressed. Appropriate trans-acting factors either are supplied by the host, supplied by a complementing vector or supplied by the vector itself upon introduction into the host.

In certain embodiments in this regard, the vectors provide for specific expression. For production of mutant $\beta_2$m, such specific expression may be inducible expression or expression only in certain types of cells or both inducible and cell-specific. Particularly preferred among inducible vectors are vectors that can be induced for expression by environmental factors that are easy to manipulate, such as temperature and nutrient additives. A variety of vectors suitable to this aspect of the invention, including constitutive and inducible expression vectors for use in prokaryotic and eukaryotic hosts, are well known and employed routinely by those of skill in the art.

A great variety of expression vectors can be used to express mutant $\beta_2$m for use in the invention. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids, all may be used for expression in accordance with this aspect of the present invention. Generally, any vector suitable to maintain, propagate or express polynucleotides to express a polypeptide in a host may be used for expression in this regard.

The appropriate DNA sequence may be inserted into the vector by any of a variety of well-known and routine techniques, such as, for example, those set forth in Sambrook et al., MOLECULAR CLONING, A LABORATORY MANUAL, 2nd Ed.; Cold Spring Harbor Laboratory Press, Cold Spring Harbor, New York (1989).

The nucleic acid sequence in the expression vector may be operatively linked to appropriate expression control sequence(s), including, for instance, a promoter to direct mRNA transcription. Representatives of such promoters include, but are not limited to, the phage lambda PL promoter, the *E. coli* lac, trp and tac promoters, for recombinant expression, and the SV40 early and late promoters and promoters of retroviral LTRs for *in situ* expression.

In general, expression constructs will contain sites for transcription initiation and termination, and, in the transcribed region, a ribosome binding site for translation. The coding portion of the mature transcripts expressed by the constructs will include a translation initiating AUG at the beginning and a termination codon appropriately positioned at the end of the polypeptide to be translated.

In addition, the constructs may contain control regions that regulate as well as engender expression. Generally, in accordance with many commonly-practised procedures, such regions will operate by controlling transcription, such as transcription factors, repressor binding sites and termination, among others.

Vectors for propagation and expression generally will include selectable markers and amplification regions, such as, for example, those set forth in Sambrook et al., MOLECULAR CLONING, A LABORATORY MANUAL, 2nd Ed.; Cold Spring Harbor Laboratory Press, Cold Spring Harbor, New York (1989).

Representative examples of appropriate hosts for recombinant expression of mutant $\beta_2$m include bacterial cells, such as *streptococci*, *staphylococci*, *E. coli*, *streptomyces* and *Bacillus subtilis* cells; fungal cells, such as yeast cells and *Aspergillus* cells; insect cells such as *Drosophila* S2 and *Spodoptera* Sf9 cells; animal or human cells such as CHO, COS, HeLa, C127, 3T3, BHK, 293 and Bowes melanoma cells; and plant cells.

The following vectors, which are commercially available, are provided by way of example. Among vectors preferred for use in bacteria are pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia, and pBR322 (ATCC 37017). Among preferred eukaryotic vectors are pWLNEO, pSV2CAT, pOG44, pXT1 and pSG available from Stratagene; and pSVK3, pBPV, pMSG and pSVL available from Pharmacia. These vectors which can be used both for recombinant expression and for *in situ* expression are listed solely by way of illustration of the many commercially available and well known vectors that are available to those of skill in the art for use in accordance with this aspect of the present invention. It will be appreciated that any other plasmid or vector suitable for, for example, introduction, maintenance, propagation or expression of a polynucleotide or polypeptide for use in the therapy of the invention in a host may be used in this aspect of the invention.

Examples of vectors for use in this aspect of the invention include expression vectors in which the mutant $\beta_2$m cDNA sequence is inserted in a plasmid whereby gene expression is driven from the human immediate early cytomegalovirus enhancer-promoter (Foecking and Hofstetter, Cell, 45, 101-105, 1986). Such expression plasmids may contain SV40 RNA processing signals such as polyadenylation and termination signals. Expression constructs which use the CMV promoter and that are commercially available are pCDM8, pcDNA1 and derivatives, pcDNA3 and derivatives (Invitrogen). Other expression vectors available which may be used are pSVK3 and pSVL which contain the SV40 promoter and mRNA splice site and polyadenylation signals from SV40 (pSVK3) and SV40 VP1 processing signals (pSVL; vectors from Pharmacia).

Promoter regions can be selected from any desired gene using vectors that contain a reporter transcription unit lacking a promoter region, such as a chloramphenicol acetyl transferase ("CAT") transcription unit, downstream of restriction site or sites for introducing a candidate promoter fragment; i.e., a fragment that may contain a promoter. As is well known, introduction into the vector of a promoter-containing fragment at the restriction site upstream of the cat gene engenders production of CAT activity, which can be detected by standard CAT assays. Vectors suitable to this end are well known and readily available, such as pKK232-8 and pCM7. Promoters for expression of polynucleotides for use in the therapy of the present invention include not only well known and readily available promoters, but also promoters that readily may be obtained by the foregoing technique, using a reporter gene; for *in situ* expression, such a promoter should be recognised in the subject to be treated.

Among known prokaryotic promoters suitable for expression of polynucleotides and polypeptides in accordance with the therapy of the present invention are the *E. coli* lacI and lacZ and promoters, the T3 and T7 promoters, the gpt promoter, the lambda PR, PL promoters and the trp promoter.

Recombinant expression vectors will include, for example, origins of replication, a promoter preferably derived from a highly-expressed gene to direct transcription of a downstream structural sequence, and a selectable marker to permit isolation of vector containing cells after exposure to the vector.

Polynucleotides for use in the therapy of the invention, encoding the mutant or modified $\beta_2$m polypeptide generally will be inserted into the vector using standard techniques so that it is operably linked to the promoter for expression. The polynucleotide will be positioned so that the transcription start site is located appropriately 5' to a ribosome binding site. The ribosome binding site will be 5' to the AUG that initiates translation of the polypeptide to be expressed.

Generally, there will be no other open reading frames that begin with an initiation codon, usually AUG, and lie between the ribosome binding site and the initiation codon. Also, generally, there will be a translation stop codon at the end of the polypeptide and there will be a polyadenylation signal in constructs for use in eukaryotic hosts. Transcription termination signal appropriately disposed at the 3' end of the transcribed region may also be included in the polynucleotide construct.

For secretion of the translated protein into the lumen of the endoplasmic reticulum, into the periplasmic space or into the extracellular environment, appropriate secretion signals may be incorporated into the expressed polypeptide when recombinantly synthesised. These signals may be endogenous to the polypeptide or they may be heterologous signals.

The polypeptide may be expressed in a modified form, such as a fusion protein, and may include not only secretion signals but also additional heterologous functional regions. Thus, for instance, a region of additional amino acids, particularly charged amino acids, may be added to the N- or C-terminus of the polypeptide to improve stability and persistence in the host cell, during purification or during subsequent handling and storage. Also, a region may be added to the polypeptide to facilitate purification. Such regions may be removed prior to final preparation of the polypeptide. The addition of peptide moieties to polypeptides to engender secretion or excretion, to improve stability or to facilitate purification, among others, are familiar and routine techniques in the art. A preferred fusion protein comprises a heterologous region from immunolglobulin that is useful to solubilise or purify polypeptides. Cells typically then are harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well known to those skilled in the art.

Mammalian expression vectors may comprise an origin of replication, a suitable promoter and enhancer, and also any necessary ribosome binding sites, polyadenylation regions, splice donor and acceptor sites, transcriptional termination sequences, and 5' flanking non-transcribed sequences that are necessary for expression.

For preparing mutant $\beta_2$m polypeptides for use in the invention, genetically engineered host cells may be used. Introduction of a polynucleotide into the host cell can be affected by calcium phosphate transfection, DEAE-dextran mediated transfection, transvection, microinjection, cationic lipid-mediated transfection, electroporation, transduction, scrape loading, ballistic introduction, infection or other methods. Such methods are described in many standard laboratory manuals, such as Davis et al., BASIC METHODS IN MOLECULAR BIOLOGY, (1986) and Sambrook et al., MOLECULAR CLONING: A LABORATORY MANUAL, 2nd Ed., Cold Spring Harbor Laboratory Press, Cold Spring Harbor, N.Y (1989).

Mature proteins can be expressed in host cells including mammalian cells such as CHO cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook et al., MOLECULAR CLONING: A LABORATORY MANUAL, 2nd Ed., Cold Spring Harbor Laboratory Press, Cold Spring Harbor, N.Y. (1989).

The polypeptide can be recovered and purified from recombinant cell cultures by well-known methods including ammonium sulphate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding protein may be employed to regenerate active conformation when the polypeptide is denatured during isolation and or purification.

Assays for $\beta_2$m exchange both on purified MHC/peptide complexes and on the cell surface (Hyafil & Strominger, Proc Natl Acad Sci U S A 76: 5834-8 (1979); Luscher, et al. J Immunol 153: 5068-81 (1994)) can be employed to investigate the ability of mutant $\beta_2$m polypeptides to exchange into MHC/peptide complexes. However, more convenient assays, with which an initial assessment of $\beta_2$m exchange and inhibition of CD8 binding can be obtained, are envisaged. Binding of CD8 to MHC/peptide complexes can be conveniently detected by surface plasmon resonance studies, for instance on the Biacore2000 or Biacore3000 systems (Garcia, et al. Nature 384: 577-81 Issn: 0028-0836 (1996); Wyer, et al. Immunity 10: 219-225 (1999)). Thus, CD8 binding to sensor cells with immobilised MHC/peptide complexes could be measured, one cell serving as control for other sensor cells on which the MHC/peptide complex has been exposed to $\beta_2$m exchange with mutant proteins. Such analysis will provide a biophysical measurement of the degree to which the mutant $\beta_2$m proteins are capable of exchanging and inhibiting CD8 binding. The production of soluble MHC-peptide complexes is well known. Soluble MHC-peptide complexes were first obtained by cleaving the molecules of the surface of antigen presenting cells with papain (Bjorkman, et al. J Mol Biol 186: 205-10 (1985)). Although this approach provided material for crystallisation, it has, for class I molecules, in recent years been replaced by individual expression of heavy and light chain in *E. coli* followed by refolding in the presence of synthetic peptide (Gao, et al. Prot. Sci. 7: 1245-49 (1998); Gao, et al. Nature 387: 630-4 (1997); Garboczi, et al. Proc Natl Acad Sci U S A 89: 3429-33 Issn: 0027-8424 (1992); Garboczi, et al. J Mol Biol 239: 581-7 Issn: 0022-2836 (1994); Madden, et al. [published erratum appears in Cell 1994 Jan 28;76(2):following 410]. Cell 75: 693-708 Issn: 0092-8674 (1993); Reid, et al. J Exp Med 184: 2279-86 (1996); Reid, et al. FEBS Lett 383: 119-23 (1996); Smith, et al. Immunity 4: 215-28 Issn: 1074-7613 (1996); Smith, et al. Immunity 4: 203-13 Issn: 1074-7613 (1996)). This approach has several advantages over previous methods in that a better yield is obtained at a lower cost, peptide identity can be controlled very accurately, and the final product is more homogenous. Furthermore, expression of modified heavy or light chain, for instance fused to a protein tag, can be easily performed.

The inhibitory effects of mutant $\beta_2$m proteins can be tested in *in vitro* CTL assays in order to assess their inhibitory effect on T cell activation. These studies can be extended to *in vivo* analysis of the effects of the mutant $\beta_2$m proteins, by testing these in relevant animal disease models. The outcome of such studies can form the basis on which further pre-clinical studies of the immune-inhibitory effects of the mutated $\beta_2$mpolypeptides are carried out.

Medicaments in accordance with the invention will usually be supplied as part of a sterile, pharmaceutical composition which will normally include a pharmaceutically acceptable carrier. This pharmaceutical composition may be in any suitable form, (depending upon the desired method of administering it to a patient).

It may be provided in unit dosage form, will generally be provided in a sealed container and may be provided as part of a kit. Such a kit would normally (although not necessarily) include instructions for use. It may include a plurality of said unit dosage forms.

The pharmaceutical composition may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such compositions may be prepared by any method known in the art of pharmacy, for example by admixing the active ingredient with the carrier(s) or excipient(s) under sterile conditions.

Pharmaceutical compositions adapted for oral administration may be presented as discrete units such as capsules or tablets; as powders or granules; as solutions, syrups or suspensions (in aqueous or non-aqueous liquids; or as edible foams or whips; or as emulsions)

Suitable excipients for tablets or hard gelatine capsules include lactose, maize starch or derivatives thereof, stearic acid or salts thereof. Suitable excipients for use with soft gelatine capsules include for example vegetable oils, waxes, fats, semi-solid, or liquid polyols etc.

For the preparation of solutions and syrups, excipients which may be used include for example water, polyols and sugars. For the preparation of suspensions oils (e.g. vegetable oils) may be used to provide oil-in-water or water in oil suspensions.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6):318 (1986).

Pharmaceutical compositions adapted for topical administration may be formulated as ointment, creams, suspensions, lotions, powders, solutions, pastes, gels, sprays, aerosols or oils. For infections of the eye or other external tissues, for example mouth and skin, the compositions are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical compositions adapted for topical administration to the eye include eye drops wherein the active ingredient is dissolved or suspended in a suitable carrier, especially an aqueous solvent. Pharmaceutical compositions adapted for topical administration in the mouth include lozenges, pastilles and mouth washes.

Pharmaceutical compositions adapted for rectal administration may be presented as suppositories or enemas.

Pharmaceutical compositions adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists which may be generated by means of various types of metered dose pressurised aerosols, nebulizers or insufflators.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solution which may contain antioxidants, buffers, bacteriostats and solutes which render the formulation substantially isotonic with the blood of the intended recipient; and aqueous and non-aqueous sterile suspensions which may include suspending agents and thickening agents. Excipients which may be used for injectable solutions include water, alcohols, polyols, glycerine and vegetable oils, for example. The compositions may be presented in unit-dose or multi-dose containers, for example sealed ampoules and vials, and may be stored in a freeze-dried (lyophilized) condition requiring only the addition of the sterile liquid carried, for example water for injections, immediately prior to use. Extemporaneous injection solutions and suspensions may be prepared from sterile powders, granules and tablets.

The pharmaceutical compositions may contain preserving agents, solubilising agents, stabilising agents, wetting agents, emulsifiers, sweeteners, colourants, odourants, salts (substances of the present invention may themselves be provided in the form of a pharmaceutically acceptable salt), buffers, coating agents or antioxidants. They may also contain therapeutically active agents in addition to the substance of the present invention.

Dosages of the substances of the present invention can vary between wide limits, depending upon the disease or disorder to be treated, the age and condition of the individual to be treated, etc. and a physician will ultimately determine appropriate dosages to be used. The dosage may be repeated as often as appropriate. If side effects develop the amount and/or frequency of the dosage can be reduced, in accordance with normal clinical practice.

Preferred features of each aspect of the invention are as for each of the other aspects *mutatis mutandis*. The prior art documents mentioned herein are incorporated to the fullest extent permitted by law.

The invention will now be described further in the following non-limiting examples. Reference is made to the accompanying drawings in which:

Figure 1a shows the amino acid sequence of the processed human $\beta_2$m polypeptide and the DNA sequence encoding it, and Figures 1b-1 show the modifications that need to be made to this DNA sequence to introduce mutations in accordance with the invention, and the amino acid sequences of the relevant regions of resulting $\beta_2$m proteins;

Figure 2 shows a Coomassie-stained SDS-PAGE gel of the *Escherichia coli* strain BL21-DE3 (pLysS) expressing human $\beta_2$m;

Figure 3 shows a Coomassie-stained SDS-PAGE gel with purified inclusion bodies containing denatured human $\beta_2$m;

Figure 4 shows a gel filtration trace of refolded human $\beta_2$m, the correctly folded protein eluting after 220 ml;

Figure 5 shows a SDS-PAGE gel with size markers in Lane 1 and refolded $\beta_2$m in Lane 2;

Figure 6 shows schematically a procedure for determining the ability of $\beta_2$m mutant proteins to exchange and to inhibit cytotoxic lymphocyte activation;

Figures 7a-e show BIAcore responses showing the ability of FLU-HLA-A2/$\beta_2$m complexes containing mutated $\beta_2$m to bind soluble CD8$\alpha\alpha$ compared to that of complexes containing wild type $\beta_2$m;

Figures 8a-e show BIAcore responses showing the ability of HLA-A2/$\beta_2$m complexes containing mutated $\beta_2$m to bind TCR compared to that of complexes containing wild type $\beta_2$m;

Figures 9a and 9b show BIAcore responses showing the ability of HLA-A2/$\beta_2$m complexes containing mutated $\beta_2$m to bind CD8 and TCR respectively after storage for 24 hours, compared to that of complexes containing wild type $\beta_2$m;

Figure 10 is a plasmid map of the plasmid pIRES;

Figure 11 shows the coding sequence of HLA-A*02011, together with primer sequences used in the present invention;

Figure 12 shows the coding sequence for full length wild type $\beta_2$m;

Figure 13 is a graph illustrating the inhibition of HLA-A2 restricted CTL clone 5D8 by mutant HLA/$\beta_2$m complexes;

Figure 14 is a graph illustrating the inhibition of HLA-A2 restricted parvovirus B19 specific CTL line by mutant HLA/$\beta_2$m complexes; and

Figure 15 shows schematically a procedure for investigating human $\beta_2$m inhibition of CTL priming *in vivo*.

Examples

*Example 1 - $K_{58} \rightarrow E$ mutation of human $\beta_2$m*

This example describes the construction of the DNA expression plasmid pEX052 which codes for the $\beta_2$m in which Lysine$_{58}$ is substituted for Glutamate. The DNA sequences of the primers used are shown highlighted in Figure 1b (K58 → E); the sequence on top corresponds to the sense strand of $\beta_2$m, the sequence on the bottom corresponds to the non-sense strand of $\beta_2$m. Mutated bases are indicated in small letters and the amino acid sequence is indicated below the DNA sequences.
A DNA plasmid, pBJ192, which encodes hu-

man $\beta_2$m in which the signal peptide is substituted for a single Methionine residue in order to allow initiation of translation when expressed in bacteria, was generated as follows. A PCR reaction was performed on cDNA generated from a human B cell line with the primers 5'- GGG GGG CAT ATG ATt CAa aGa ACT CCA Aaa ATT CAG GTT TAC TCA CGT CAT CC -3' (forward primer) and 5'- GGG GGA AGC TTA CAT GTC TCG ATC CCA CTT AAC TAT - 3' (backward primer). Bases shown in small letters indicate mutations in relation to the human sequence, These do not alter the sequence of the polypeptide that is encoded, in relation to the human sequence, but were introduced in order to increase expression of the $\beta_2$m protein in *E. coli*. The PCR product was cloned into pGMT7 (Studier, et al. Methods Enzymol 185: 60-89 Issn: 0076-6879 (1990)) at the restriction sites for NdeI and HindIII.
100 ng of plasmid pBJ192 was mixed with 5 $\mu$l 10 mM dNTP, 25 $\mu$l 10xPfu-buffer (Stratagene), 10 units Pfu polymerase (Stratagene) and the final volume was adjusted to 240 $\mu$l with H$_2$O. 48 $\mu$l of this mix was supplemented with primers diluted to give a final concentration of 0.2 $\mu$M in 50 $\mu$l final reaction volume. After an initial denaturation step of 30 seconds at 95° the reaction mixture was subjected to 15 rounds of denaturation (95°C, 30 sec.), annealing (55°C, 60 sec.), and elongation (73°C, 8 min.) in a Hybaid PCR express PCR machine. The product was then digested for 5 hours at 37°C with 10 units of DpnI restriction enzyme (New England Biolabs). 10 $\mu$l of the digested reaction was transformed into XL1-Blue bacteria and grown for 18 hours at 37°C. A single colony was picked and grown over night in 5 ml TYP + ampicillin (16 g/l Bacto-Tryptone, 16 g/l Yeast Extract, 5 g/l NaCl, 2.5 g/l K$_2$HPO$_4$, 100 mg/l Ampicillin). Plasmid DNA was purified on a Qiagen mini-prep column according to the manufacturer's instructions and the sequence was verified by automated sequencing at the sequencing facility of Department of Biochemistry, Oxford University.

*Example 2 - $K_{58} \rightarrow S$ mutation of human $\beta_2$m*

This example describes the construction of the DNA expression plasmid pEX053 which codes for the $\beta_2$m in which Lysine$_{58}$ is substituted for Serine. The DNA sequences of the primers used are shown in Figure 1c (K58 → S). Mutation of DNA plasmid pBJ192 was carried out according to the same protocol as described in Example 1 and the sequence was verified by automated sequencing at the sequencing facility of Department of Biochemistry, Oxford University.

*Example 3 - $K_{58} \rightarrow R$ mutation of human $\beta_2$m*

This example describes the construction of the DNA expression plasmid pEX051 which codes for the $\beta_2$m in which Lysine$_{58}$ is substituted for Arginine. The DNA sequences of the primers used are shown in Figure 1d (K58 → R). Mutation of DNA plasmid pEX052 was carried out according to the same protocol as described in Example 1 except that 20 PCR rounds were carried out and the elongation time was increased to 10 minutes. The sequence was verified by automated sequencing at the sequencing facility of Department of Biochemistry, Oxford University.

*Example 4 - $K_{58} \to Y$ mutation of human $\beta_2 m$*

This example describes the construction of the DNA expression plasmid pEX054 which codes for the $\beta_2 m$ in which Lysine$_{58}$ is substituted for Tyrosine. The DNA sequences of the primers used are shown in Figure le (K58 → Y). Mutation of DNA plasmid pEX052 was carried out according to the same protocol as described in Example 3 and the sequence was verified by automated sequencing at the sequencing facility of Department of Biochemistry, Oxford University.

*Example 5 - $K_{58} \to C$ mutation of human $\beta_2 m$*

This example describes the construction of the DNA expression plasmid pEX055 which codes for the $\beta_2 m$ in which Lysine$_{58}$ is substituted for Cysteine. The DNA sequences of the primers used are shown in Figure 1f (K58 → C). Mutation of DNA plasmid pEX052 was carried out according to the same protocol as described in Example 3 and the sequence is verified by automated sequencing at the sequencing facility of Department of Biochemistry, Oxford University.

*Example 6 - $K_{58} \to SES$ mutation of human $\beta_2 m$*

This example describes the construction of the DNA expression plasmid pEX056 which codes for the $\beta_2 m$ in which Lysine$_{58}$ is substituted for Serine-Glutamate-Serine. The DNA sequences of the primers used are shown in Figure 1g (K58 → SES). Mutation of DNA plasmid pEX052 was carried out according to the same protocol as described in Example 3 and the sequence was verified by automated sequencing at the sequencing facility of Department of Biochemistry, Oxford University.

*Example 7 - $K_{58} \to W$ mutation of human $\beta_2 m$*

This example describes the construction of the DNA expression plasmid pEX061 which codes for the $\beta_2 m$ in which Lysine$_{58}$ is substituted for Tryptophan. The DNA sequences of the primers used are shown in Figure 1h (K58 → W). Mutation of DNA plasmid pEX051 was carried out according to the following protocol.

10 ng of template plasmid (pEX051) was mixed with 1.25 $\mu$l 10 mM dNTP, 5 $\mu$l 10xPfu-buffer (Stratagene), 2.5 units Pfu polymerase (Stratagene) and the final volume was adjusted to 50 $\mu$l with H$_2$O. 48 $\mu$l of this mix was supplemented with primers diluted to give a final concentration of 0.2 $\mu$M in 50 $\mu$l final reaction volume.

After an initial denaturation step of 2 minutes at 95°C the reaction mixture was subjected to 18 rounds of denaturation (95°C, 30 sec.), annealing (55°C, 60 sec.), and elongation (68°C, 10 min.) in a Hybaid PCR express PCR machine. The product was then digested for 1 hour at 37°C with 20 units of DpnI restriction enzyme (New England Biolabs). 10 $\mu$l of the digested reaction was transformed into XL1-Blue bacteria and grown for 18 hours at 37°C. A single colony was picked and grown over night in 5 ml TYP + ampicillin (16 g/l Bacto-Tryptone, 16 g/l Yeast Extract, 5 g/l NaCl, 2.5 g/l K$_2$HPO$_4$,100 mg/l Ampicillin). Plasmid DNA was purified on a Qiagen Spin miniprep column according to the manufacturer's instructions and the sequence was verified by automated sequencing at the sequencing facility of Department of Biochemistry, Oxford University.

*Example 8 - $K_{58} \to GRG$ mutation of human $\beta_2 m$*

This example describes the construction of the DNA expression plasmid pEX062 which codes for the $\beta_2 m$ in which Lysine$_{58}$ is substituted for the tri-peptide Glycine-Arginine-Glycine. The DNA sequences of the primers used are shown in Figure 1i (K58 → GRG). Mutation of DNA plasmid pEX056 was carried out according to the same protocol as described in Example 7 and the sequence was verified by automated sequencing at the sequencing facility of Department of Biochemistry, Oxford University.

*Example 9 - $D_{59} \to GEG$ mutation of human $\beta_2 m$*

This example describes the construction of the DNA expression plasmid pEX063 which codes for the $\beta_2 m$ in which Aspartate$_{59}$ is substituted for the tri-peptide Glycine-Glutamate-Glycine. The DNA sequences of the primers used are shown in Figure 1j (D59 → GEG). Mutation of DNA plasmid pEX050 was carried out according to the same protocol as described in Example 7 and the sequence was verified by automated sequencing at the sequencing facility of Department of Biochemistry, Oxford University.

*Example 10 - $W_{60} \to G$ mutation of human $\beta_2 m$*

This example describes the construction of the DNA expression plasmid pEX064 which codes for the $\beta_2 m$ in which Tryptophan$_{60}$ is substituted for Glycine. The DNA sequences of the primers used are shown in Figure 1k (W60 → Y). Mutation of DNA plasmid pEX050 was carried out according to the same protocol as described in Example 7 and the sequence was verified by automated sequencing at the sequencing facility of Department of Biochemistry, Oxford University.

*Example 11 - $K_{58} W_{60} \to RG$ mutation of human $\beta_2 m$*

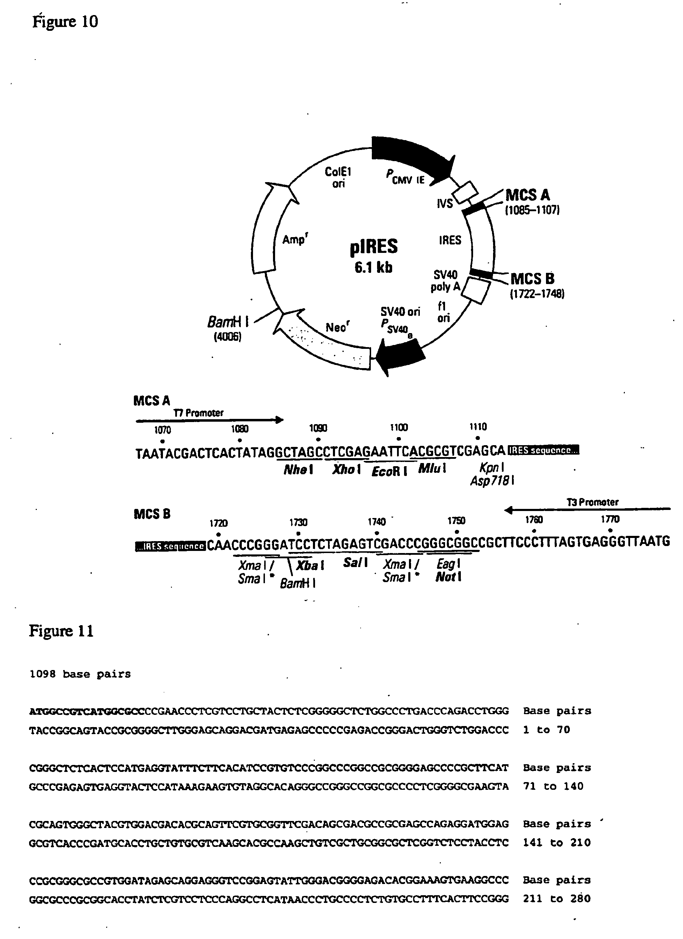

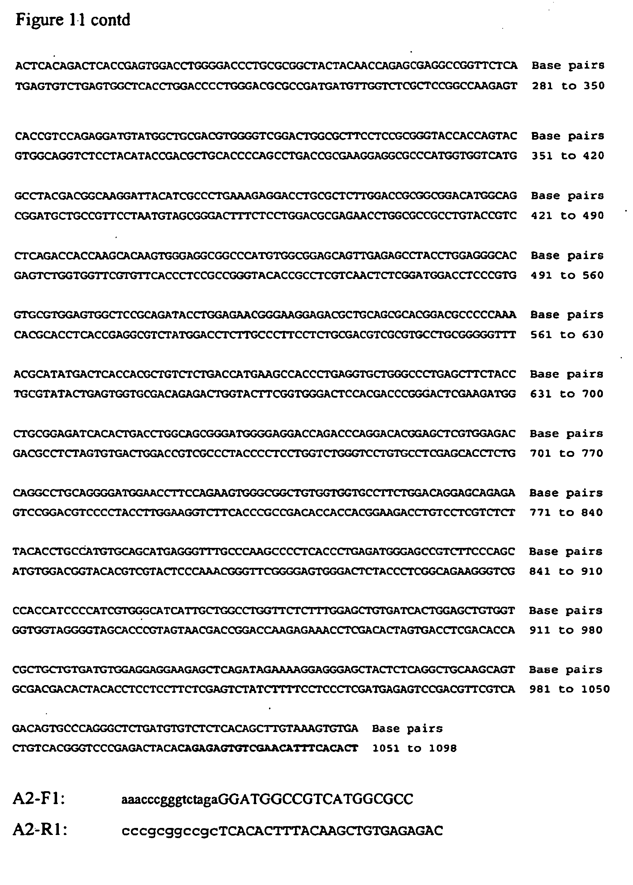

This example describes the construction of the DNA expression plasmid pEX065 which codes for the $\beta_2$m in which Lysine$_{58}$ and Tryptophan$_{60}$ are substituted for Arginine and Glycine respectively. The DNA sequences of the primers used are shown in Figure 11 (K58W60 → RG). Mutation of DNA plasmid pEX051 was carried out according to the same protocol as described in Example 7 and the sequence was verified by automated sequencing at the sequencing facility of Department of Biochemistry, Oxford University.

*Example 12 - Expression, refolding and purification of human $\beta_2$m and $\beta_2$m mutants*

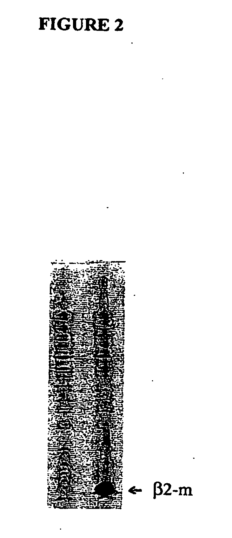

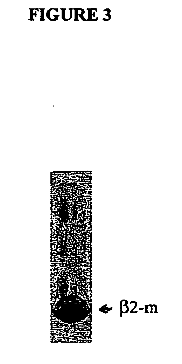

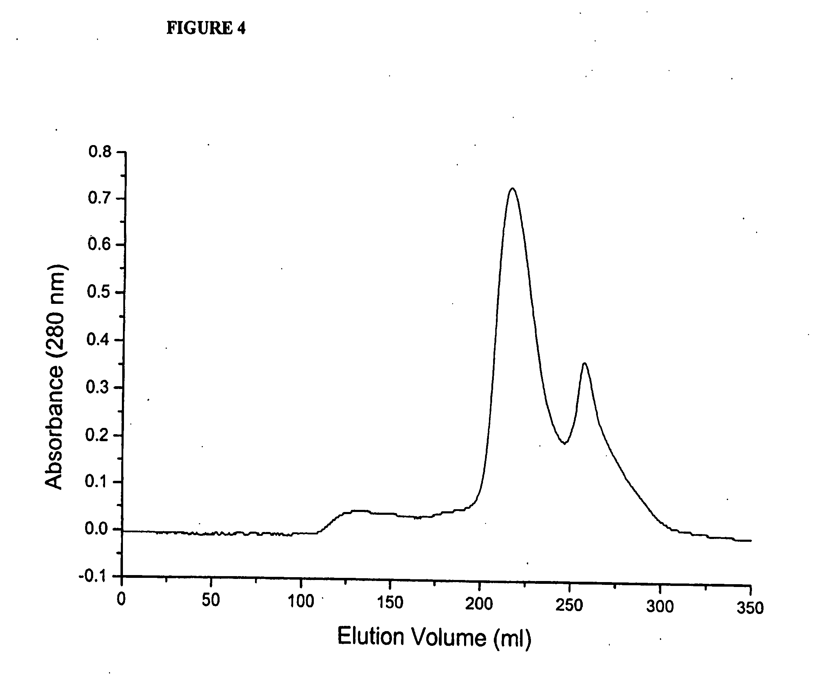

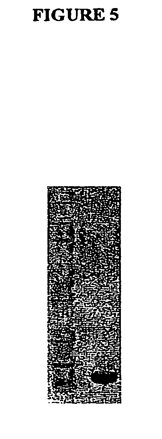

$\beta_2$m protein was expressed from the DNA vector pBJ192, and from mutated derivatives of pBJ192, e.g. pEX051-56 and pEX061-65, in the *Eschericia coli* strain BL21-DE3 pLysS (Novagen). pBJ192 contains the $\beta_2$m gene under the control of the strongly inducible T7 promoter in the vector pGMT7 (Studier, et al. Methods Enzymol 185: 60-89 Issn: 0076-6879 (1990)). The BL21 cells transformed with one of the $\beta_2$m-expressing vectors were plated on LB/agar/100 mg/ml ampicillin plates made according to a standard recipe. Transformants were then grown in TYP medium with Ampicillin (16 g/l Bacto-Tryptone, 16 g/l Yeast Extract, 5 g/l NaCl, 2.5 g/l K$_2$HPO$_4$, 100 mg/l Ampicillin) to an OD$_{600}$ ~ 0.4. For large-scale expression, 1 l volumes of TYP media were prepared in 21 conical flasks and were covered with four layers of aluminium foil and were autoclaved. Cell densities were measured using optical density at 600 nm wavelength (OD600) on a Beckman DU530 spectrophotometer. Sterile TYP media was used as a blank. Figure 2 shows a Coomassie-stained SDS-PAGE gel of the *Escherichia coli* strain BL21-DE3 (pLysS) expressing human $\beta_2$m. Lane 1 contains a bacterial extract before induction of T7 transcription, lane 2 shows an extract from the same bacterial culture, but after 3 hours of induction of T7 transcription. Prior to induction, very little $\beta_2$m was produced by the bacteria, but after induction with 0.5 mM Iso-phenyl thio-galactoside (IPTG, from Melford), large amounts of $\beta_2$m were produced and deposited in inclusion bodies. Inclusion bodies were purified as described (Gao, et al, Prot. Sci.7: 1245-49 (1998)). Cells were lysed in 'Lysis Buffer' (10 mM EDTA (from 0.5 M stock pH 8.0), 2 mM DTT (from 1 M stock in 10 mM sodium acetate pH 5.2, stored at -20°C), 10 mM Tris pH 8.1 (from 2 M stock pH 8.1), 150 mM NaCl (from 4 M stock), 200 $\mu$g/ml lysozyme (from 20 mg/ml stock stored at -20°C), 10% glycerol (from fluid), 2500 units of DNAase I and 10mM MgCl$_2$ using a 50 ml Dounce homogeniser DNase I and lysozyme were from Sigma). Sonication, in lysis buffer, to break open the cells was performed using a 12mM probe sonicator (Milsonix XL2020). The probe was tuned according to the manufacturers instructions. The resulting suspension was then diluted 1:1 in 'Triton Buffer' (0.5% (w/v) Triton X-100 (from fluid), 50 mM Tris pH 8.1 (from 2 M stock), 100 mM NaCl (from 4 M stock), 0.1% sodium azide (from solid), 10 mM EDTA (from 0.5 M stock pH 8.0), 2 mM DTT (from 1 M stock in 10 mM sodium acetate pH 5.2, stored at -20°C) and left overnight. The inclusion bodies were separated from cell debris by centrifugation in a Beckman J2-21 centrifuge equipped with a JA-20 rotor as described (Gao, et al, Prot. Sci.7: 1245-49 (1998)) and stored at -20°C. Inclusion bodies were then thawed and resuspended in 'Resuspension Buffer' (50 mM Tris pH 8.1 (from 2 M stock), 100 mM NaCl (from 4 M stock), 10 mM EDTA (from 0.5 M stock pH 8.0), 2 mM DTT (from 1 M stock in 10 mM sodium acetate pH 5.2, stored at -20°C)), and denatured in 6M Guanidine and 10mM DTT buffered with Tris-HCl pH 8.1 (all chemicals from Sigma). Figure 3 shows a Coomassie-stained SDS-PAGE gel with purified inclusion bodies containing denatured human $\beta_2$m. $\beta_2$m is then refolded *in vitro* in the presence of 0.4M L-Arginine and purified by gel filtration chromotography, for instance on a Pharmacia Superdex 75 column. Figure 4 shows a gel filtration trace of refolded human $\beta_2$m, the correctly folded protein eluting after 220 ml. $\beta_2$m protein typically expresses at a level >100 mg/l medium and refolds at an efficiency of ~36% giving high yields of correctly refolded soluble protein. It elutes from the Superdex 75 PG (preparation grade 26/60) column (Pharmacia) at an elution volume of 220 ml. It was judged, by Coomassie-stained SDS-PAGE, to be >95% pure after the single purification step. Figure 5 shows a SDS-PAGE gel with size markers in Lane 1 and refolded $\beta_2$m in Lane 2.

*Example 13 - Testing of CD8 binding to HLA complex incorporating $\beta_2$m mutant proteins*

$\beta_2$m mutant proteins were prepared according to Example 12, using the plasmids described in Examples 1-11. After the purification of denatured $\beta_2$m mutant protein, refolding was carried out in the presence of peptide and denatured HLA heavy chain (Gao, et al, Prot. Sci.7: 1245-49 (1998)) containing a tag sequence that can be enzymatically biotinylated (Schatz, Biotechnology N Y 11: 1138-43 (1993); Altman, et al Science 274: 94-6 (1996); Wyer, et al. Immunity 10: 219-225 (1999)). The complex was then biotinylated using the enzyme BirA (O'Callaghan, et al. Anal Biochem 266(1): 9-15 (1999)) to produce mutant $\beta_2$m- HLA-A2 - peptide complexes which were biotinylated towards the C-terminus of the HLA-A2 heavy chain. These protein complexes were immobilised on a streptavidin-modified BIAcore chip sensor cell in a BIAcore 3000 machine. The binding of soluble CD8$\alpha\alpha$ were monitored by surface plasmon resonance when the CD8 protein was caused to flow over the sensor cell at a concentration in the range of 10-20 mg/ml.

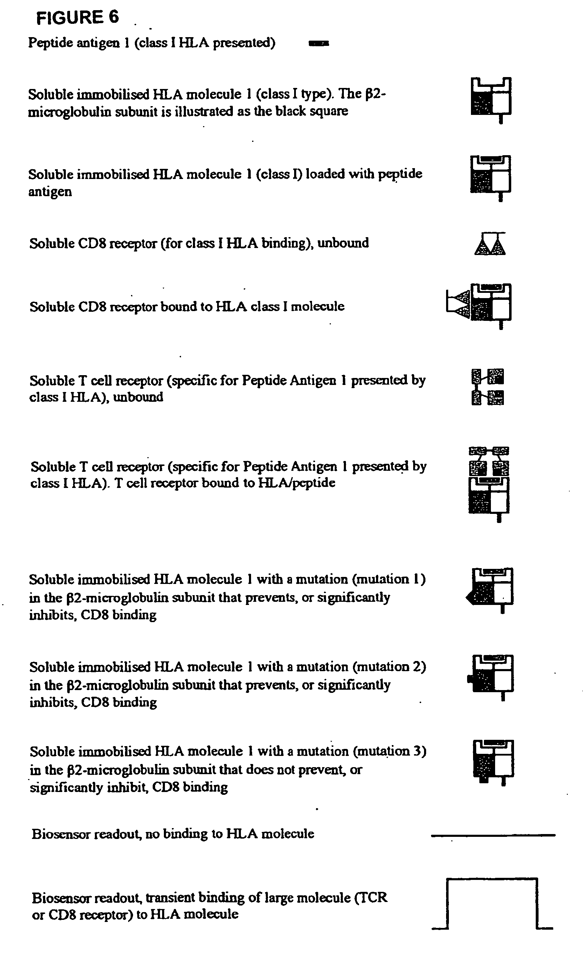

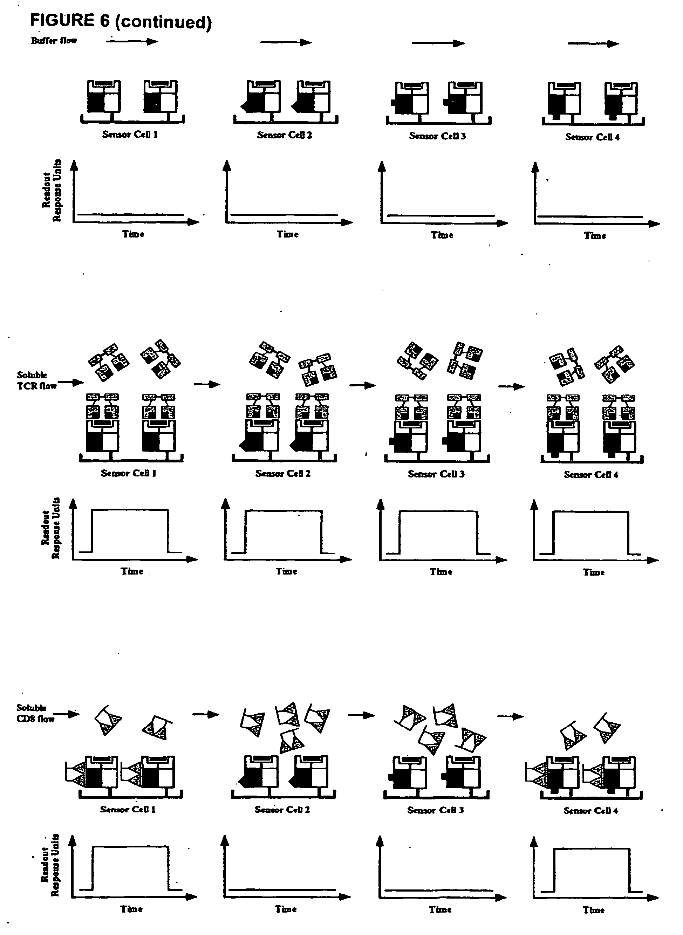

Determination of the effects on CD8 binding of mutations introduced in the $\beta_2$m protein was accomplished with an experimental set up as illustrated in Figure 6. The HLA (or MHC) complex involving non-mutated $\beta_2$m protein was immobilised in one sensor cell, the HLA complexes involving each mutated $\beta_2$m protein was immobilised in each other sensor cell. To test that all complexes were correctly folded, soluble TCR, specific for the HLA/peptide complex used, was passed through all the sensor cells used (Wyer, et al. Immunity 10: 219-225 (1999)). Detection of the antigen specific interaction is an extremely strong indication that folding of the HLA complex is correct. Next, soluble CD8 protein was passed over all of the sensor cells and the levels of binding in these were compared. If binding of soluble CD8 in a sensor cell was absent, or significantly reduced, then it was concluded that the mutant $\beta_2$m protein incorporated into this complex had affected CD8's ability to bind the complex.

The following $\beta_2$m mutants were prepared.

| Mutation | Plasmid |
|---|---|
| 58K → R | pEX 051 |
| 58K→ E | pEX 052 |
| 58K→S | pEX 053 |
| 58K → Y | pEX 054 |
| 58K→ C | pEX 55 |
| 58K→ SES | pEX 056 |
| 58K→ W | pEX061 |
| 58K→ GRG | pEX062 |
| 59D→ GEG | pEX063 |
| 60W→ G | pEX064 |
| 59K/60W→ R/G | pEX065 |

The ability of HLA-A2/$\beta_2$m complexes containing the above mutations to bind soluble CD8$\alpha\alpha$ was compared to that of complexes containing WT $\beta_2$m, as described above.

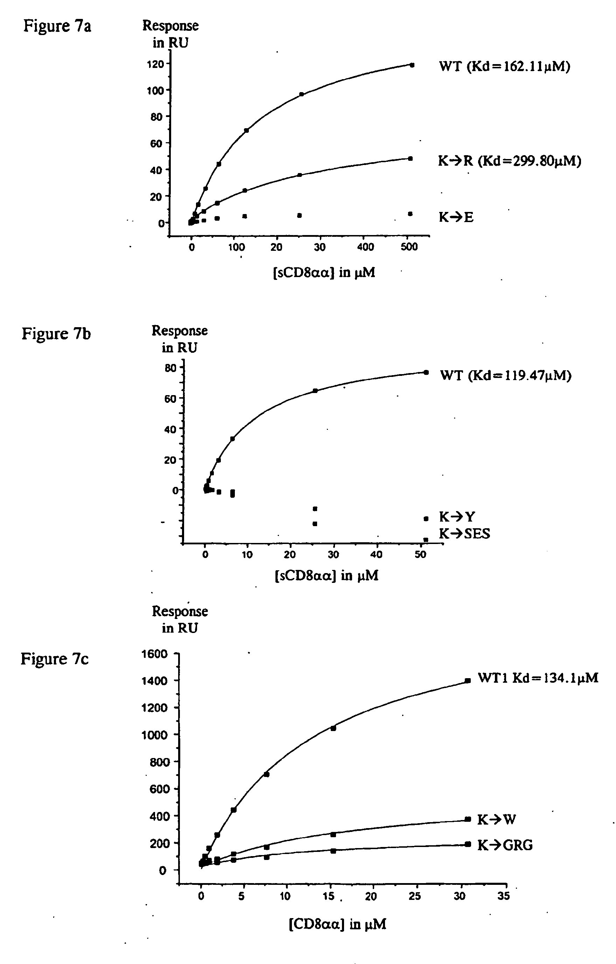

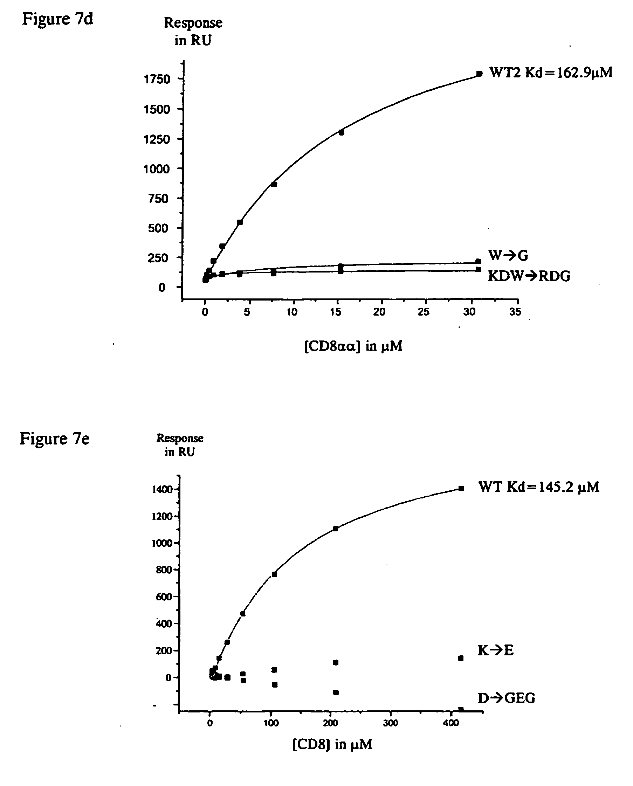

The results of studies to determine the binding of HLA-A2/mutant $\beta_2$m complexes to bind soluble CD8$\alpha\alpha$ are shown in Figures 7a-e.

The concentration in $\mu$M is calculated from the measured absorbance of the sCD8$\alpha\alpha$ stock solution (Conc = Absx10$^6$/(extinct co x Mw)). The response is the difference between the response obtained for one cell containing a complex and the reference cell and is indicated in Response Units. The concentration of sCD8$\alpha\alpha$ in $\mu$M was plotted (x) against the response in RU (y) and the points were fitted in the equation:

$$Response = n[CD8]/([CD8] + Kd).$$

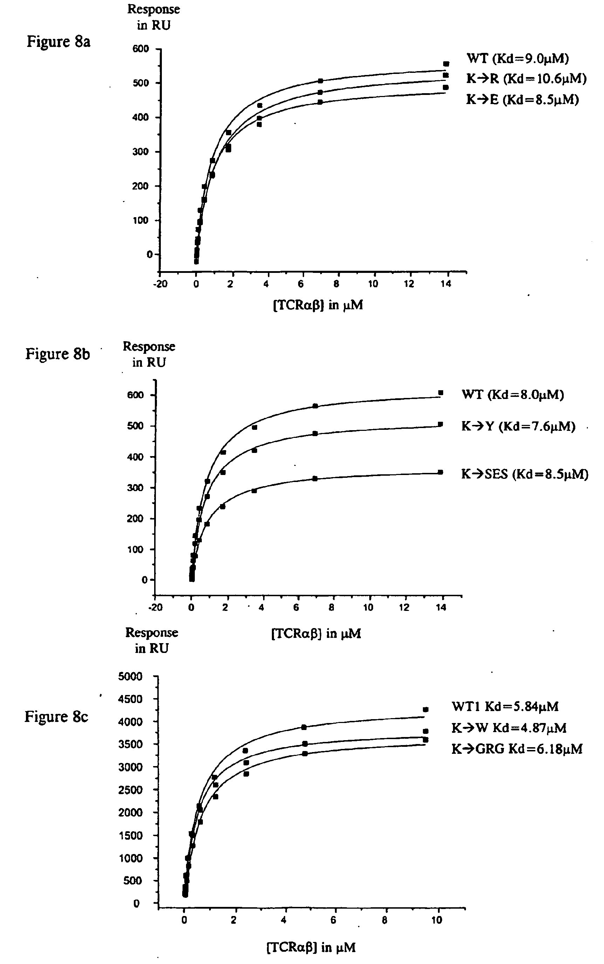

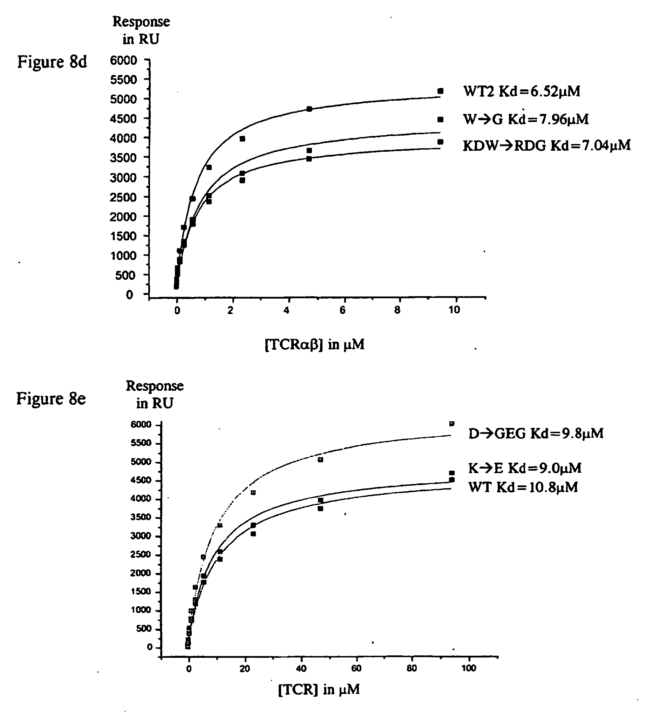

The ability of the HLA/mutant $\beta_2$m complexes to be conveniently refolded and to bind to the appropriate TCR, were also investigated. The results are shown in Figures 8a-e. The concentration of TCR $\mu$M was plotted (x) against the response in RU (y) and the points were fitted in the equation Response = n[TCR]/([TCR] + Kd).

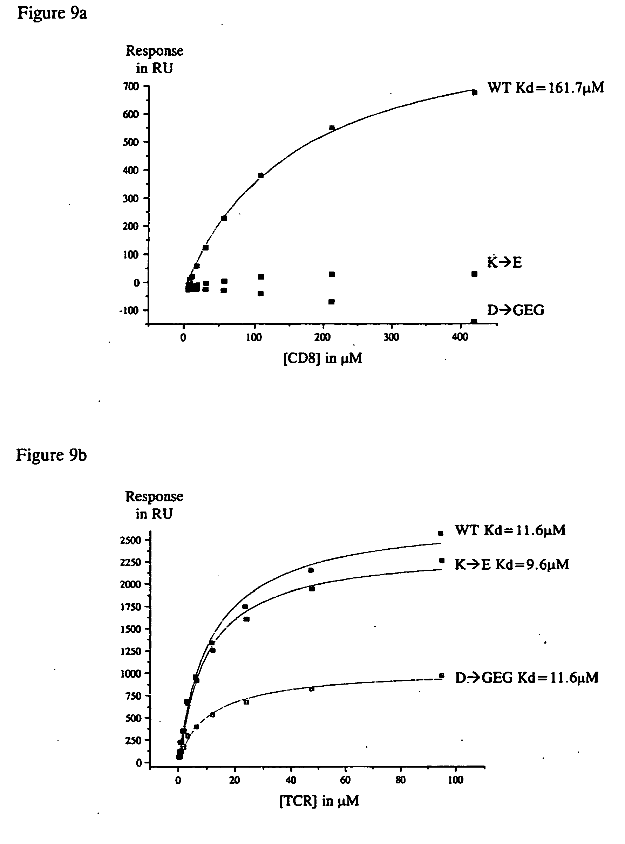

The stability of the HLA-A2-mutant $\beta_2$m complexes was also investigated for the 58K→ E and 59D → GEG mutants by re-assessing their ability to bind CD8 and TCR after storage at room temperature on a BIA core chip for 24 hours. The results are shown in Figures 9a and 9b respectively. The 24 hour stability assessment demonstrated no change in the CD8 or TCR binding response for the 58K→ E mutant. However the TCR binding of the 59D→ GEG mutant demonstrated a drop in total binding response with a practically unchanged Kd.

The results obtained (see Figures 7a-e) demonstrate that the HLA-A2 $\beta_2$m substitution mutations cause a decrease in affinity for soluble CD8$\alpha\alpha$. Almost total inhibition of CD8 binding was caused by the following mutations:

K58→SES, K58→E, K58→Y, W60→G, K58/W60→R/G, K58→W, D59→GEG and K58→GRG.

Partial inhibition of CD8 binding was caused by the following mutations, in order of decreasing inhibition:

K58→R > K58→C > K58→S.

The recovery on refolding of the mutants was also assessed (see Figures 8a-e) and the mutants are listed in order of decreasing recovery:

K58→R = K58→E > K58→Y = K58→W > K58→C = K58→S > K58→SES = D59→GEG = K58→GRG > W60→G = K58/W60→R/G

The ability of the mutants to bind TCR was also assessed (see Figures 8a-e) and the mutants tested are listed in order of decreasing total binding response:

K58→R = D59→GEG = K58→E > K58→Y = K58→W > K58→SES = K58→GRG = W60→G = K58/W60→R/G (K58→C & K58→S not tested)

Among the mutants that totally inhibit CD8 binding, only K58→E refolds as well as WT $\beta$2m and is fully functional. The 24 hour storage tests indicate that the K58→E mutant is stable over this time period whereas the D59→GEG mutant undergoes partial degradation.

*Example 14 - Compounds which may be attached to Cysteine in the $\beta_2$m $K_{58} \to$ C. mutant and linkers which may effect the attachment.*

Mutation of Lysine$_{58}$ in $\beta_2$m to a Cysteine residue allows the unpaired SH group of the Cysteine to be used to link other compounds to the mutated $\beta_2$m protein. Such other compounds are used sterically to hinder CD8 binding to HLA (or MHC) complexes of which the mutated and derivatised $\beta_2$m forms a part. This strategy is similar to those described above where mutation to residues with larger side chains are used to prevent or inhibit CD8 binding. The difference with using derivatisation of the SH group of Cysteine at position 58 is that relatively larger compounds can be incorporated into the protein. This can cause a more efficient steric hindrance than can be achieved with non-derivatised amino acid mutations. However, this involves a further step in the preparation and may reduce the ability of the protein to exchange into HLA/peptide complexes.

Compounds that can be used to derivatise $\beta_2$m $K_{58} \rightarrow C$ include amino acids, peptides, Biotin, and DNA, all of which would be expected to severely affect CD8's ability to slot into its binding site on HLA (or MHC) complexes.

Derivatisation is performed with a suitable linker molecule, such as Maleimide, Iodoacetamide, 3,-(pyridyldithio)propionate and 3,-(pyridyldithio)propionamide, all of which are commercially available from chemical reagent companies (Sigma, Aldrich, Pierce). These linker compounds can be used to attach an almost limitless variety of groups to a Cysteine residue, either by producing a linker-derivatised molecule (e.g. 3-(N-Maleimidopropionyl) Biocytin™ available from Sigma) or by using a heterobifunctional molecule such as e-Maleimidocaproic acid N-hydroxysuccinimide ester (Sigma) to conjugate e.g. a protein.

*Example 15 - HLA/mutant $\beta$2m mammalian expression vector for immunosuppressive therapy*

There are a number of expression systems, known to those skilled in the art, which can be appropriate for the therapeutic delivery of the proteins of the present invention. DNA can be delivered by a number of different established routes using viral vectors, injection of naked DNA into the recipient tissue in a formulation optimised for cellular uptake, injection using accelerated DNA coated particles, or any technology designed for *in vivo* gene delivery.

HLA/mutant $\beta$2m complexes can be administered against CTL based immune disorders using modified adeno-associated virus. This is a vector which can be used for the therapeutic delivery of DNA, and is capable of infecting a wide rage of tissue types and therefore could be used for a variety of applications.

Tissue specificity of expression can be controlled by using tissue specific promoter and enhancer sequences in conjunction with a general mode of delivery. Tissue specificity can also be obtained by using viral vectors which only infect certain cell or tissue types in conjunction with using general regulatory elements controlling the expression of the genes. The vector and regulatory sequence combination of choice will depend on the nature of the disease being treated.

The human creatine kinase (CKM) promotor is an example of a tissue-specific regulatory sequence capable of targeted DNA expression in muscle tissue. The human cytomegalovirus (CMV) promoter is an example of a regulatory sequence capable of inducing DNA expression in a broad range of tissues.

*Example 16 - Production of HLA-$\beta$2m mammalian expression vector for in vitro T cell assays*

This example details the protocol used to produce an appropriate bicistronic vector for the production of wild type (WT) and HLA-mutant $\beta$2m expression vectors, which can be used to induce expression of these proteins for *in-vitro* T cell assays.

$10^7$ T2 cells (Payne et al, Immunogenetics (1990) 31(3):169-78) cells were harvested and lysed in 1 ml of TRI reagent from Sigma and total RNA was harvested according to instructions provided by the manufacturer. 10 $\mu$g total RNA, 0.01 OD$_{260}$ of d(pT)$_{12\text{-}18}$ primer, 10 $\mu$l of 10xRT buffer and 5 $\mu$l Omniscript Reverse Transcriptase (Qiagen Omniscript RT-kit Cat. No. 205111 lot EGQ002) was used for cDNA synthesis in a total volume of 100 $\mu$l according to instructions provided by the manufacturer. PCR was performed on 0.5 $\mu$l T2 cDNA in a total volume 40 $\mu$l using the primers A2-F1 (aaacccgggtctagaggatggccgtcatggcc cc) and A2-R1 (cccgcggccgctcacactttacaagctgtgagagac) at 0.5 $\mu$M each, 0.2 mM dNTP, and 2 units of cloned Pfu DNA polymerase (Stratagene # 600153). The programme used an initial 10 minutes denaturation step at 95°C followed by 30 cycles of denaturation for 1 minute at 95°C, annealing for 1 min at 50°C, and elongation for 4 minutes at 73°C and a final elongation step at 73°C. The product was purified after electrophoresis on a 1 % agarose gel by electro-transfer to GF/C filter, elution from the filter by centrifugation, extraction by Phenol:Chloroform:Isoamylic alcohol (25:24:1) and chromatograhy purified on a Superdex G50 Spincolumn. The purified PCR product and 200 ng pIRES (Clontech #6028-1) was digested with 10 units XbaI and 5 units Not I in a final volume of 20 $\mu$l Tris-Acetate buffer (33 mM Tris Acetate pH 7.9, 66 KOAc, 10mM MgOAc, 0.1 mg/ml autoclaved gelatin, 0.5 mM DTT). Digests were purified from an 0.9% agarose/TBE gel using the same technique as described above. The fragment was ligated into pIRES (see Figure 10) using a Rapid DNA Ligation Kit (Roche # 1 635 379) according to the manufacturers instructions. The entire HLA-A*0201 coding sequence (see Figure 11) was cloned between the XmaI or the XbaI site and the NotI site in MCS B. The design of the oligo-nucleotides allows amplification of this sequence from human cDNA. The insert sequence of the resulting plasmid, pEX060, was verified by automated sequencing.

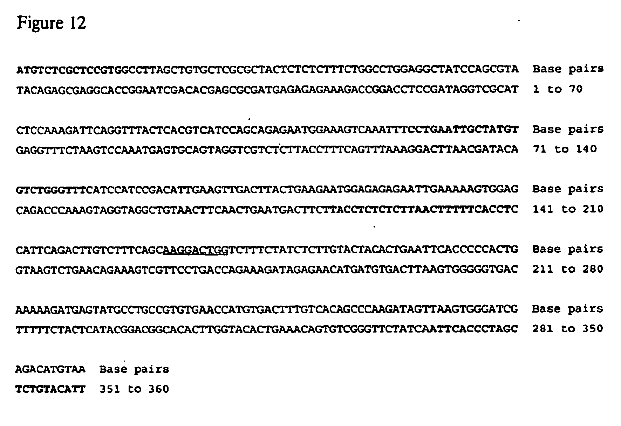

The gene coding for full length wild type $\beta$2m (see Figure 12) was amplified from total T cell RNA by RT-PCR using primers $\beta$2m-F2 (cccagctagctcgagatgtctcgctccgtggct) and $\beta$2m-R3 (aaacacgcgttacatgtctcgatcccactta) and cloned XhoI-MluI into pIRES by standard techniques as described above. The insert sequence of the resulting plasmid, pEX066, was verified by automated sequencing.

Different genes coding for full length mutant $\beta$2m proteins were prepared by PCR-stitching using primers $\beta$2m-F2 + $\beta$2m-R2 (tccactttttcaattctctctccatt) on

T cell cDNA template for the 5'-end and primers β2m-F3 (cctgaattgctatgtgtctgggtt) and β2m-R3 on plasmid pEX051, pEX052, or pEX56 as template for the 3'-end followed by PCR amplification of the mixed products using β2m-F2 and β2m-R3 and cloned Xho I - Mlu I into pIRES by standard techniques as described above. The insert sequences of the resulting plasmids, pEX067 (K58→E), pEX068 (K58→SES), and pEX069 (K58→R) were verified by automated sequencing.

The HLA-A*02011 fragment from pEX060 was subcloned by standard techniques Xba I - Not I into each of plasmids pEX066, pEX067, pEX068, and pEX069 to give bi-cistronic expression plasmids pEX070 (wt. + HLA-A*02011), pEX071(K58→E + HLA-A*02011), pEX072 (K58→SES + HLA-A*02011), and pEX073(K58→R + HLA-A*02011) respectively.

The vector can be used to induce transient expression of HLA-A*0201 combined with wild type- or mutant β2m in appropriate human target cells. The transfected target cell can be any HLA-A*0201 negative Antigen presenting cell (APC), for example, C1R, T293 or HeLa cells. Transfected and peptide labelled cells can be used as targets for lysis by HLA-A*0201 specific CTLs in a standard chromium release assay, or similar.

*Example 17 - Assay for Immune-inhibitory effect of HLA-mutant β2m complexes*

The ability of target cells transfected with HLA-mutant β2m complexes as described in Example 16 to inhibit *in vitro* CTL activity is determined by the following methodology.

A human T cell line (enriched and selected), recognising the target HLA e.g. HLA-A*0201 is used. A constant number of target cells (5000), expressing the HLA-A*0201-β2m complex, are used and the number of T cells are varied. 'E:T' represents a ratio of numbers of T cells to target cells. Four E:T ratios are tested. The peptide concentrations are varied from $10^{-11}$ to $10^{-7}$ M. Two sets of experiments probing the mutant HLA-A*0201-mutant β2m complex effect are conducted as follows:

> Group 1 - incubation with target cells expressing only the WT HLA-A*0201-β2m complex as a control;
> Group 2 - incubation with target cells expressing HLA-A2*0201-mutant -β2m complex.

The assay components for these experiments are: 18 μl 10X peptide; 18 μl PBS; 50 μl containing 5000 target cells; 100 μl containing 5000 -50000 CTL. Results are collected after 2 hours incubation.

The inhibition of CTL activity in cells expressing the HLA A2*0201-mutant -β2m complexes indicates their immune-suppressive activity.

*Example 18 - Inhibition of HLA-A2 restricted CTL clone 5D8 by HLA/ mutant β2m complexes*

Target cells (T2 hybrids) were grown in RPMI containing 10% human serum for 5 days. These cells were then incubated with wild-type or mutant β2m protein (K58→R, K58→Y, K58→E, K58→SES) at 300μg/ml in RPMI medium containing 1μM peptide (SLYNTVATL, GAG P17-HIV1) for 2 hours. These cells were then washed and labelled with $^{51}$Cr for 1 hour in the presence of 150μg/ml wild-type or mutant β2m protein. After further washes, the target cells were plated out with CTL at a range of E:T ratios. Wild-type or mutant β2m protein was present at 300μg/ml for the duration of the assay. Supernatants were harvested after 2 hours.

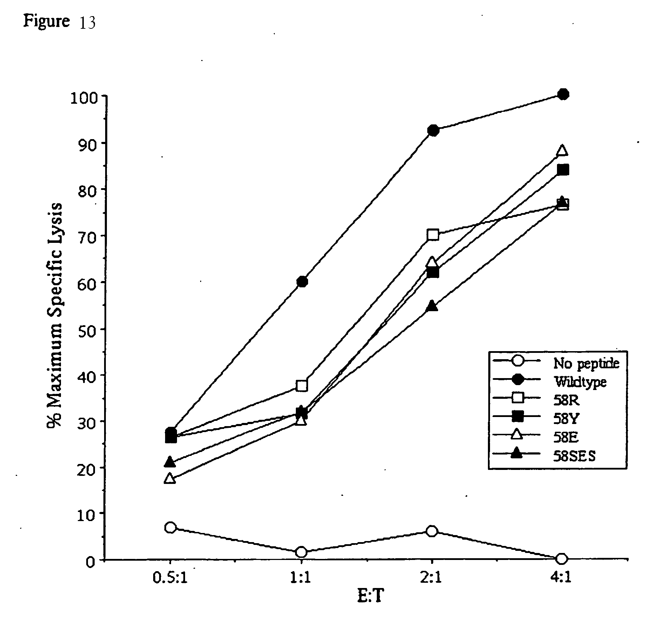

The results are shown in Figure 13, in which it can be seen that all of the mutants tested exhibited an inhibition of CTL activity at E:T ratios of 1:1 or greater.

*Example 19 - Inhibition of HLA-A2 restricted parvovirus B19 specific CTL line by HLA/mutant β2m complexes*

Target cells (T2 hybrids) were grown in RPMI containing 10% bovine serum for 5 days. These cells were then washed and labelled with $^{51}$Cr for 1 hour. The target cells were then incubated with either wild-type β2m or mutant β2m (K58→E) at 30μg/ml in RPMI medium containing 100 nM peptide (epitope is EADVQQWLTW) for 15 minutes. The target cells were plated out with CTL (CTL line) at a ratio of 22:1 (E:T ratio). Wild-type (WT) β2m or mutant β2m was present at 30μg/ml for the duration of the assay. Supernatants were harvested after 4 hours.

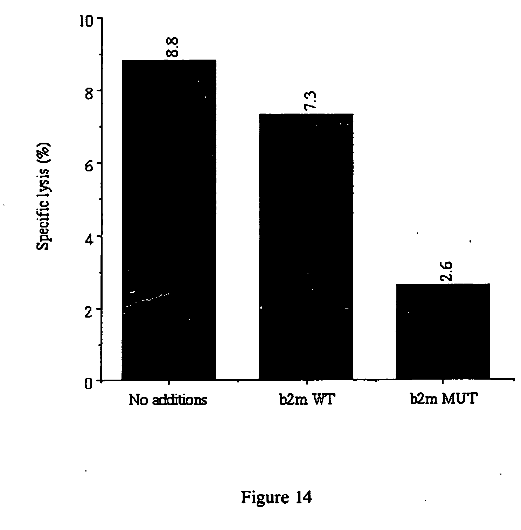

The results are shown in Figure 14. The addition of mutant β2m protein resulted in a specific lysis of 2.6% compared to 7.3% in the presence of WT β2m protein. This demonstrates the ability of mutant β2m protein to inhibit CTL activity.

*Example 20 - Test for Immunogenicity of mutant β-2-microglobulin*

Mutant β-2-microglobulin molecules could potentially induce an immune response either by antibodies and/or T cells. The introduction of mutation(s) in the β-2-microglobulin protein could potentially introduce a conformational change in the protein structure, which would be recognised by antibodies as a structural foreign antigen or, alternatively, enzymatic degradation of mutant β-2-microglobulin could produce peptides not normally presented by self MHC molecules. These mutant peptides would then be recognised as foreign and induce a cellular immune response. Therefore, mutants can be tested in a transgenic rat model expressing human MHC class I molecule (HLA-B27 heavy chain + β-2-microglobulin) as follows.

> 1. Inject transgenic rats with 3-4mg mutant beta-2-microglobulin.

2. Collect serum from rats after 21 days.

3. Analyse serum for the production of anti-mutant β-2-microglobulin antibodies by an ELISA. The procedure for this ELISA is: a. Bind mutant beta-2-microglobulin to bottom of well; b. Add serum from transgenic rat, which has been treated with mutant beta-2-microglobulin; c. wash three times with 200μL of wash buffer; d.add the appropriate concentration of conjugated anti-rat antibody; e. wash three times with 200μL of wash buffer; f. add 100μL detection reagent (Alkaline Phosphatase, substrate pNPP) and read absorbance at 405nm. Absorbance readings above negative control readings will indicate, that anti-mutant β-2-microglobulin antibodies are present in the serum.

*Example 21 - Inhibition of CTL priming and activity by mutant HLA/β2m complexes in vivo.*

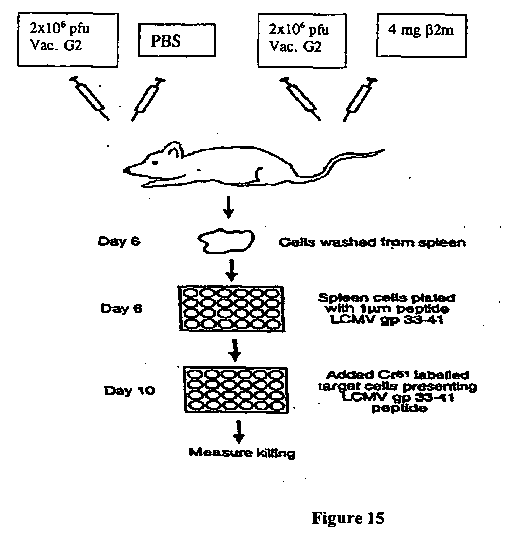

The ability of mutant β2m to inhibit a cellular immune response *in vivo* is tested by three experiments in mouse, assaying the influence of mutant β2m on the response to an epitope encoded in a vaccinia virus (See Figure 15).

Materials and reagents:

Mice: In all three experiments, mice of strain Black 6 (C57BL/6 wildtype laboratory mouse strain) are used.

Virus: Vaccinia virus strain G2 (vvG2) is a recombinant virus strain. Infection of cells with Vaccinia virus generally results in the MHC restricted presentation of a number of vaccinia peptide epitopes which are poorly characterised. Infection with strain vvG2, however, also results in the presentation of a peptide epitope, corresponding to amino acids 33-41 (sequence in one-letter code: KAVYNFATC) from the LCM virus G2 glycoprotein. In mice, the G2 epitope is presented by the MHC molecule D[b].

Target cells : MC57 antigen presenting cells (Leist, et al (1987) J Immunol 138: 2278-81) express mouse MHC molecules K[b] and D[b].

Experiment 1:

A control group of mice are injected, intravenously, at Day 0 with 2x10[6] pfu (plaque forming units) vaccinia virus strain G2 plus 200 μl phosphate buffered saline (PBS, standard physiological saline buffer). A test group of mice are also injected, intravenously, at Day 0 with 2x10[6] pfu Vaccinia virus strain G2 plus 200 μl mutant β2m protein (20mg/ml) in PBS buffer.

To assay CTL activity, a modification of a previously described procedure can be used (Leist, et al (1987) J Immunol 138: 2278-81). On Day 6, the spleens are removed from the mice and lymphocytes washed out. Spleen cells are cultured in 24-well plates. Synthetic peptide corresponding to the G2 33-41 epitope is added to the wells and lymphocytes allowed to proliferate for four days. On Day 10, T cells are counted and CTL killing assays are performed as follows. Chromium[51] -labelled MC57 cells are prepulsed with G2 33-41 peptide for one hour, then mixed with the cultured mouse lymphocytes at a range of effector to target ratios. Chromium[51]. counts from wells of MC57 cells lysed by addition of detergent are set at 100% lysis.

Experiment 2:

The control and test group of mice are treated identically to those in Experiment 1 except that injections are performed intraperitoneally. CTL activity is assessed as described in Experiment 1.

Experiment 3:

A control group of mice are injected intraperitoneally with 200 μl phosphate buffered saline. This dose is administered five hours prior to infection vaccinia virus strain G2, and a further injection 24 hours after the first one. A test group of mice is injected intraperitoneally with mutant β2m protein (4 mg) in PBS buffer. This dose is administered five hours prior to infection vaccinia virus strain G2, and a further injection 24 hours after the first one.

CTL activity is assessed as described in Experiment 1.

**Claims**

1. The use of a modified human β2-microglobulin with a mutation at one or more of Serine57, Lysine58, Aspartate59, Tryptophan60 and Serine61 whose binding to CD8 is inhibited in the manufacture of a medicament for inhibiting CD8[+] T cell response.

2. The use as claimed in claim 1, wherein the modified human β2-microglobulin is formulated for administration extracellularly as a soluble recombinant protein or intracellularly by transfection of a DNA construct encoding the modified human β2-microglobulin.

3. The use as claimed in claim 1 or claim 2, wherein the mutated residue is mutated to Cysteine which is derivatised.

4. The use as claimed in claim 1 or claim 2, wherein Lysine58 is replaced with Arginine, Glutamate, Serine, Tyrosine, Tryptophan, Cysteine, Serine-Glutamate-Serine, or Glycine-Arginine-Glycine.

5. The use as claimed in claim 1 or claim 2, wherein Lysine58 is replaced with Glutamate, Arginine or Aspartate.

6. The use as claimed in any one of claims 1, 2, 4 or 5, wherein Aspartate$_{59}$ is replaced with Glycine-Glutamate-Glycine.

7. The use as claimed in any one of claims 1, 2, 4, 5 or 6, wherein Tryptophan$_{60}$ is replaced with Glycine.

8. The use as claimed in any one of claims 1, 2, 4 or 5, wherein Lysine$_{58}$ is replaced with Arginine, and Tryptophan$_{60}$ is replaced with Glycine.

9. The use as claimed in claim 1 or claim 2, wherein, when Aspartate$_{59}$ is mutated to Alanine, Phenylalanine$_{56}$ is not mutated to Tyrosine; when Tryptophan$_{60}$ is mutated to Arginine, Leucine$_{54}$ is not mutated to Valine and/or Leucine$_{64}$ is not mutated to Arginine; when Tryptophan$_{60}$ is mutated to Cysteine, another residue is also mutated; when Tryptophan$_{60}$ is mutated to Leucine, Histidine$_{51}$ is not mutated to Tyrosine; and when Tryptophan$_{60}$ is mutated to Glycine, Aspartate$_{53}$ is not mutated to Tyrosine.

10. The use as claimed in any preceding claim for the treatment of autoimmune disorder, graft versus host disease, or graft rejection.

11. A modified human $\beta_2$m, wherein:

Lysine$_{58}$ is replaced with Serine, Tyrosine, Tryptophan, Cysteine, Serine-Glutamate-Serine, or Glycine-Arginine-Glycine; or
Aspartate$_{59}$ is replaced with Glycine-Glutamate-Glycine; or
Lysine$_{58}$ is replaced with Arginine and Tryptophan$_{60}$ is replaced with Glycine.

**Patentansprüche**

1. Verwendung eines modifizierten humanen $\beta_2$-Mikroglobulins ($\beta_2$m) mit einer Mutation an einem oder mehreren von Serin$_{57}$, Lysin$_{58}$, Aspartat$_{59}$, Tryptophan$_{60}$ und Serin$_{61}$, dessen Bindung an CD8 inhibiert ist, zur Herstellung eines Medikaments zur Inhibierung der CD8$^+$ T-Zellantwort.

2. Verwendung nach Anspruch 1, wobei das modifizierte humane $\beta_2$-Mikroglobulin zur extrazellulären Verabreichung als lösliches rekombinantes Protein oder zur intrazellulären Verabreichung durch Transfektion eines DNA-Konstrukts, das für das modifizierte humane $\beta_2$-Mikroglobulin codiert, formuliert ist.

3. Verwendung nach Anspruch 1 oder Anspruch 2, wobei der mutierte Rest zu Cystein mutiert ist, das derivatisiert ist.

4. Verwendung nach Anspruch 1 oder Anspruch 2, wobei Lysin$_{58}$ durch Arginin, Glutamat, Serin, Tyrosin, Tryptophan, Cystein, Serin-Glutamat-Serin oder Glycin-Arginin-Glycin ersetzt ist.

5. Verwendung nach Anspruch 1 oder Anspruch 2, wobei Lysin$_{58}$ durch Glutamat, Arginin oder Aspartat ersetzt ist.

6. Verwendung nach einem der Ansprüche 1, 2, 4 oder 5, wobei Aspartat$_{59}$ durch Glycin-Glutamat-Glycin ersetzt ist.

7. Verwendung nach einem der Ansprüche 1, 2, 4, 5 oder 6, wobei Tryptophan$_{60}$ durch Glycin ersetzt ist.

8. Verwendung nach einem der Ansprüche 1, 2, 4 oder 5, wobei Lysin$_{58}$ durch Arginin, und Tryptophan$_{60}$ durch Glycin ersetzt ist.

9. Verwendung nach Anspruch 1 oder Anspruch 2, wobei
wenn Aspartat$_{59}$ zu Alanin mutiert ist, Phenylalanin$_{56}$ nicht zu Tyrosin mutiert ist;
wenn Tryptophan$_{60}$ zu Arginin mutiert ist, Leucin$_{54}$ nicht zu Valin mutiert ist und/oder Leucin$_{64}$ nicht zu Arginin mutiert ist;
wenn Tryptophan$_{60}$ zu Cystein mutiert ist, ein anderer Rest ebenso mutiert ist;
wenn Tryptophan$_{60}$ zu Leucin mutiert ist, Histidin$_{51}$ nicht zu Tyrosin mutiert ist; und
wenn Tryptophan$_{60}$ zu Glycin mutiert ist, Aspartat$_{53}$ nicht zu Tyrosin mutiert ist.

10. Verwendung nach einem vorhergehenden Anspruch zur behandlung einer Autoimmunerkrankung, Transplantat-versus-Wirt-Krankheit oder Transplantatabstoßung.

11. Modifiziertes humanes $\beta_2$m, wobei
Lysin$_{58}$ durch Serin, Tyrosin, Tryptophan, Cystein, Serin-Glutamat-Serin oder Glycin-Arginin-Glycin ersetzt ist; oder
Aspartat$_{59}$ durch Glycin-Glutamat-Glycin ersetzt ist; oder
Lysin$_{58}$ durch Arginin ersetzt ist und Tryptophan$_{60}$ durch Glycin ersetzt ist.

**Revendications**

1. Utilisation d'une $\beta_2$-microglobuline humaine modifiée par une mutation d'un ou plusieurs acides aminés parmi Sérine$_{57}$, Lysine$_{58}$, Aspartate$_{59}$, Tryptophane$_{60}$ et Sérine$_{61}$ dont la liaison au CD8 est inhibée dans la fabrication d'un médicament pour inhiber la réponse des lymphocytes CD8$^+$ T.

2. Utilisation selon la revendication 1, dans laquelle la $\beta_2$-microglobuline humaine modifiée est formulée pour administration par voie extracellulaire sous forme d'une protéine recombinante soluble ou par voie intracellulaire par transfection d'une structure ADN codant pour la $\beta_2$-microglobuline humaine modifiée.

3. Utilisation selon la revendication 1 ou la revendication 2, dans laquelle le résidu muté est muté en Cystéine, qui est dérivée.

4. Utilisation selon la revendication 1 ou la revendication 2, dans laquelle Lysine$_{58}$ est remplacé par Arginine, Glutamate, Sérine, Tyrosine, Tryptophane, Cystéine, Sérine-Glutamate-Sérine ou Glycine-Arginine-Glycine.

5. Utilisation selon la revendication 1 ou la revendication 2, dans laquelle Lysine$_{58}$ est remplacé par Glutamate, Arginine ou Aspartate.

6. Utilisation selon l'une quelconque des revendications 1, 2, 4 ou 5, dans laquelle Aspartate$_{59}$ est remplacé par Glycine-Glutamate-Glycine.

7. Utilisation selon l'une quelconque des revendications 1, 2, 4, 5 ou 6, dans laquelle Tryptophane$_{60}$ est remplacé par Glycine.

8. Utilisation selon l'une quelconque des revendications 1, 2, 4 ou 5, dans laquelle Lysine$_{58}$ est remplacé par Arginine et Tryptophane$_{60}$ est remplacé par Glycine.

9. Utilisation selon la revendication 1 ou 2, dans laquelle
   quand Aspartate$_{59}$ est muté en Alanine, Phénylalanine$_{56}$ n'est pas muté en Tyrosine;
   quand Tryptophane$_{60}$ est muté en Arginine, Leucine$_{54}$ n'est pas muté en Valine et/ou Leucine$_{64}$ n'est pas muté en Arginine;
   quand Tryptophane$_{60}$ est muté en Cystéine, un autre résidu est également muté;
   quand Tryptophane$_{60}$ est muté en Leucine, Histidine$_{51}$ n'est pas muté en Tyrosine; et
   quand Tryptophane$_{60}$ est muté en Glycine, Aspartate$_{53}$ n'est pas muté en Tyrosine.

10. Utilisation selon l'une quelconque des revendications précédentes, pour le traitement d'une maladie auto-immune, d'une réaction du greffon contre l'hôte ou d'un rejet de greffe.

11. $\beta_2$-m humaine modifiée, dans laquelle :

Lysine$_{58}$ est remplacé par Sérine, Tyrosine, Tryptophane, Cystéine, Sérine-Glutamate-Sérine, ou Glycine-Arginine-Glycine; ou
Aspartate$_{59}$ est remplacé par Glycine-Glutamate-Glycine; ou
Lysine$_{58}$ est remplacé par Arginine et Tryptophane$_{60}$ est remplacé par Glycine.

## Figure 1a

```
atcATCCAGCGTACTCCAAAGATTCAGGTTTACTCACGTCATCCAGCAGAGAATGGAAAGTCAAATTTCCTGA Base
tacTAGGTCGCATGAGGTTTCTAAGTCCAAATGAGTGCAGTAGGTCGTCTCTTACCTTTCAGTTTAAAGGACT  70
   M  I  Q  R  T  P  K  I  Q  V  Y  S  R  H  P  A  E  N  G  K  S  N  F  L  N
      1                          10                         20

ATTGCTATGTGTCTGGGTTTCATCCATCCGACATTGAAGTTGACTTACTGAAGAATGGAGAGAGAATTGA  Base
TAACGATACACAGACCCAAAGTAGGTAGGCTGTAACTTCAACTGAATGACTTCTTACCTCTCTCTTAACT  140
   C  Y  V  S  G  F  H  P  S  D  I  E  V  D  L  L  K  N  G  E  R  I  E
         30                            40

AAAAGTGGAGCATTCAGACTTGTCTTTCAGCAAGGACTGGTCTTTCTATCTCTTGTACTACACTGAATTC  Base
TTTTCACCTCGTAAGTCTGAACAGAAAGTCGTTCCTGACCAGAAAGATAGAGAACATGATGTGACTTAAG  210
   K  V  E  H  S  D  L  S  F  S  K  D  W  S  F  Y  L  L  Y  Y  T  E  F
      50                            60                            70

ACCCCCACTGAAAAAGATGAGTATGCCTGCCGTGTGAACCATGTGACTTTGTCACAGCCCAAGATAGTTA  Base
TGGGGGTGACTTTTTCTACTCATACGGACGGCACACTTGGTACACTGAAACAGTGTCGGGTTCTATCAAT  280
   T  P  T  E  K  D  E  Y  A  C  R  V  N  H  V  T  L  S  Q  P  K  I  V  K
                           80                            90

AGTGGGATCGAGACATGTAA  Base pairs
TCACCCTAGCTCTGTACATT  281 to 300
   W  D  R  D  M  *
```

## Figure 1b: K58 → E

```
                              ΔNruI
AAAAGTGGAGCATTCAGACTTGTCTTTCtctgagGACTGGTCTTTCTATCTCTTGTACTACACTGAATTC  Base
TTTTCACCTCGTAAGTCTGAACAGAAAGagactcCTGACCAGAAAGATAGAGAACATGATGTGACTTAAG  210
   K  V  E  H  S  D  L  S  F  S  E  D  W  S  F  Y  L  L  Y  Y  T  E  F
      50                            60                            70
```

## Figure 1c: K58 → S

```
                              ΔNruI
AAAAGTGGAGCATTCAGACTTGTCTTTCtcttctGACTGGTCTTTCTATCTCTTGTACTACACTGAATTC  Base
TTTTCACCTCGTAAGTCTGAACAGAAAGagaagaCTGACCAGAAAGATAGAGAACATGATGTGACTTAAG  210
   K  V  E  H  S  D  L  S  F  S  S  D  W  S  F  Y  L  L  Y  Y  T  E  F
      50                            60                            70
```

## Figure 1d: K58 → R

```
                              ΔNruI
AAAAGTGGAGCATTCAGACTTGTCTTTCtctcgcGACTGGTCTTTCTATCTCTTGTACTACACTGAATTC  Base
TTTTCACCTCGTAAGTCTGAACAGAAAGagagcgCTGACCAGAAAGATAGAGAACATGATGTGACTTAAG  210
   K  V  E  H  S  D  L  S  F  S  R  D  W  S  F  Y  L  L  Y  Y  T  E  F
      50                            60                            70
```

## Figure 1e: K58 → Y

```
                              ΔNruI
AAAAGTGGAGCATTCAGACTTGTCTTTCtcttacGACTGGTCTTTCTATCTCTTGTACTACACTGAATTC  Base
TTTTCACCTCGTAAGTCTGAACAGAAAGagaatgCTGACCAGAAAGATAGAGAACATGATGTGACTTAAG  210
   K  V  E  H  S  D  L  S  F  S  Y  D  W  S  F  Y  L  L  Y  Y  T  E  F
      50                            60                            70
```

## Figure 1f: <u>K58 → C</u>

ΔNruI

```
AAAAGTGGAGCATTCAGACTTGTCTTTCAGCtgtGACTGGTCTTTCTATCTCTTGTACTACACTGAATTC  Base
TTTTCACCTCGTAAGTCTGAACAGAAAGTCGacaCTGACCAGAAAGATAGAGAACATGATGTGACTTAAG  210
  K   V   E   H   S   D   L   S   F   S   C   D   W   S   F   Y   L   L   Y   Y   T   E   F
          50                              60                             70
```

## Figure 1g: <u>K58 → SES</u>

```
AAAAGTGGAGCATTCAGACTTGTCTTTCAGCtctgagtctGACTGGTCTTTCTATCTCTTGTACTACACTGAATTC  Base
TTTTCACCTCGTAAGTCTGAACAGAAAGTCGagactcagaCTGACCAGAAAGATAGAGAACATGATGTGACTTAAG  210
  K   V   E   H   S   D   L   S   F   S   E   S   D   W   S   F   Y   L   L   Y   Y   T   E   F
```

## Figure 1h: <u>K58 → W</u>

```
AAAAGTGGAGCATTCAGACTTGTCTTTCTCTtggGACTGGTCTTTCTATCTCTTGTACTACACTGAATTC  Base
TTTTCACCTCGTAAGTCTGAACAGAAAGAGAaccCTGACCAGAAAGATAGAGAACATGATGTGACTTAAG  210
  K   V   E   H   S   D   L   S   F   S   W   D   W   S   F   Y   L   L   Y   Y   T   E   F
```

## Figure 1i: <u>K58→GRG</u>

```
AAAAGTGGAGCATTCAGACTTGTCTTTCTCTggtcgcggcGACTGGTCTTTCTATCTCTTGTACTACACTGAATTC  Base
TTTTCACCTCGTAAGTCTGAACAGAAAGAGAccagcgccgCTGACCAGAAAGATAGAGAACATGATGTGACTTAAG  210
  K   V   E   H   S   D   L   S   F   S   G   R   G   D   W   S   F   Y   L   L   Y   Y   T   E   F
```

## Figure 1j: <u>D59→GEG</u>

```
AAAAGTGGAGCATTCAGACTTGTCTTTCAGCAAGggtgagggcTGGTCTTTCTATCTCTTGTACTACACTGAATTC  Base
TTTTCACCTCGTAAGTCTGAACAGAAAGTCGTTCccactcccgACCAGAAAGATAGAGAACATGATGTGACTTAAG  210
  K   V   E   H   S   D   L   S   F   S   K   G   E   G   W   S   F   Y   L   L   Y   Y   T   E   F
```

## Figure 1k: <u>W60→ G</u>

```
AAAAGTGGAGCATTCAGACTTGTCTTTCAGCAAGGACggtTCTTTCTATCTCTTGTACTACACTGAATTC  Base
TTTTCACCTCGTAAGTCTGAACAGAAAGTCGTTCCTGccaAGAAAGATAGAGAACATGATGTGACTTAAG  210
  K   V   E   H   S   D   L   S   F   S   K   D   G   S   F   Y   L   L   Y   Y   T   E   F
```

## Figure 1l: <u>K58W60→RG</u>

```
AAAAGTGGAGCATTCAGACTTGTCTTTCTCTCGCGACggtTCTTTCTATCTCTTGTACTACACTGAATTC  Base
TTTTCACCTCGTAAGTCTGAACAGAAAGAGAGCGCTGccaAGAAAGATAGAGAACATGATGTGACTTAAG  210
  K   V   E   H   S   D   L   S   F   S   R   D   G   S   F   Y   L   L   Y   Y   T   E   F
```

**FIGURE 2**

← β2-m

**FIGURE 3**

← β2-m

FIGURE 4

Absorbance (280 nm)

Elution Volume (ml)

**FIGURE 5**

## FIGURE 6

Peptide antigen 1 (class I HLA presented)    ▬

Soluble immobilised HLA molecule 1 (class I type). The β2-microglobulin subunit is illustrated as the black square

Soluble immobilised HLA molecule 1 (class I) loaded with peptide antigen

Soluble CD8 receptor (for class I HLA binding), unbound

Soluble CD8 receptor bound to HLA class I molecule

Soluble T cell receptor (specific for Peptide Antigen 1 presented by class I HLA), unbound

Soluble T cell receptor (specific for Peptide Antigen 1 presented by class I HLA). T cell receptor bound to HLA/peptide

Soluble immobilised HLA molecule 1 with a mutation (mutation 1) in the β2-microglobulin subunit that prevents, or significantly inhibits, CD8 binding

Soluble immobilised HLA molecule 1 with a mutation (mutation 2) in the β2-microglobulin subunit that prevents, or significantly inhibits, CD8 binding

Soluble immobilised HLA molecule 1 with a mutation (mutation 3) in the β2-microglobulin subunit that does not prevent, or significantly inhibit, CD8 binding

Biosensor readout, no binding to HLA molecule

Biosensor readout, transient binding of large molecule (TCR or CD8 receptor) to HLA molecule

## FIGURE 6 (continued)

Buffer flow

Sensor Cell 1    Sensor Cell 2    Sensor Cell 3    Sensor Cell 4

Readout Response Units / Time

Soluble TCR flow

Sensor Cell 1    Sensor Cell 2    Sensor Cell 3    Sensor Cell 4

Readout Response Units / Time

Soluble CD8 flow

Sensor Cell 1    Sensor Cell 2    Sensor Cell 3    Sensor Cell 4

Readout Response Units / Time

Figure 7a

Figure 7b

Figure 7c

Figure 7d

Figure 7e

Figure 8a

Response in RU

WT (Kd=9.0μM)
K→R (Kd=10.6μM)
K→E (Kd=8.5μM)

[TCRαβ] in μM

Figure 8b

Response in RU

WT (Kd=8.0μM)

K→Y (Kd=7.6μM)

K→SES (Kd=8.5μM)

[TCRαβ] in μM

Figure 8c

Response in RU

WT1 Kd=5.84μM
K→W Kd=4.87μM
K→GRG Kd=6.18μM

[TCRαβ] in μM

Figure 8d

Response in RU

WT2 Kd=6.52µM

W→G Kd=7.96µM

KDW→RDG Kd=7.04µM

[TCRαβ] in µM

Figure 8e

Response in RU

D→GEG Kd=9.8µM

K→E Kd=9.0µM
WT Kd=10.8µM

[TCR] in µM

Figure 9a

Figure 9b

## Figure 10

MCS A

T7 Promoter

1070      1080      1090      1100      1110

TAATACGACTCACTATAGGCTAGCCTCGAGAATTCACGCGTCGAGCA IRES sequence...

Nhe I   Xho I   EcoR I   Mlu I   Kpn I
                                  Asp718 I

MCS B

T3 Promoter

1720      1730      1740      1750      1760      1770

...IRES sequence CAACCCGGGATCCTCTAGAGTCGACCCGGGCGGCCGCTTCCCTTTAGTGAGGGTTAATG

Xma I /   \Xba I   Sal I   Xma I /   Eag I
Sma I *   BamH I            Sma I *   Not I

## Figure 11

1098 base pairs

```
ATGGCCGTCATGGCGCCCGAACCCTCGTCCTGCTACTCTCGGGGGCTCTGGCCCTGACCCAGACCTGGG     Base pairs
TACCGGCAGTACCGCGGGGCTTGGGAGCAGGACGATGAGAGCCCCCGAGACCGGGACTGGGTCTGGACCC     1 to 70


CGGGCTCTCACTCCATGAGGTATTTCTTCACATCCGTGTCCCGGCCCGGCCGCGGGGAGCCCCGCTTCAT     Base pairs
GCCCGAGAGTGAGGTACTCCATAAAGAAGTGTAGGCACAGGGCCGGGCCGGCGCCCCTCGGGGCGAAGTA     71 to 140


CGCAGTGGGCTACGTGGACGACACGCAGTTCGTGCGGTTCGACAGCGACGCCGCGAGCCAGAGGATGGAG     Base pairs
GCGTCACCCGATGCACCTGCTGTGCGTCAAGCACGCCAAGCTGTCGCTGCGGCGCTCGGTCTCCTACCTC     141 to 210


CCGCGGGCGCCGTGGATAGAGCAGGAGGGTCCGGAGTATTGGGACGGGGAGACACGGAAAGTGAAGGCCC     Base pairs
GGCGCCCGCGGCACCTATCTCGTCCTCCCAGGCCTCATAACCCTGCCCCTCTGTGCCTTTCACTTCCGGG     211 to 280
```

## Figure 11 contd

```
ACTCACAGACTCACCGAGTGGACCTGGGGACCCTGCGCGGCTACTACAACCAGAGCGAGGCCGGTTCTCA     Base pairs
TGAGTGTCTGAGTGGCTCACCTGGACCCCTGGGACGCGCCGATGATGTTGGTCTCGCTCCGGCCAAGAGT     281 to 350


CACCGTCCAGAGGATGTATGGCTGCGACGTGGGGTCGGACTGGCGCTTCCTCCGCGGGTACCACCAGTAC     Base pairs
GTGGCAGGTCTCCTACATACCGACGCTGCACCCCAGCCTGACCGCGAAGGAGGCGCCCATGGTGGTCATG     351 to 420


GCCTACGACGGCAAGGATTACATCGCCCTGAAAGAGGACCTGCGCTCTTGGACCGCGGCGGACATGGCAG     Base pairs
CGGATGCTGCCGTTCCTAATGTAGCGGGACTTTCTCCTGGACGCGAGAACCTGGCGCCGCCTGTACCGTC     421 to 490


CTCAGACCACCAAGCACAAGTGGGAGGCGGCCCATGTGGCGGAGCAGTTGAGAGCCTACCTGGAGGGCAC     Base pairs
GAGTCTGGTGGTTCGTGTTCACCCTCCGCCGGGTACACCGCCTCGTCAACTCTCGGATGGACCTCCCGTG     491 to 560


GTGCGTGGAGTGGCTCCGCAGATACCTGGAGAACGGGAAGGAGACGCTGCAGCGCACGGACGCCCCCAAA     Base pairs
CACGCACCTCACCGAGGCGTCTATGGACCTCTTGCCCTTCCTCTGCGACGTCGCGTGCCTGCGGGGGTTT     561 to 630


ACGCATATGACTCACCACGCTGTCTCTGACCATGAAGCCACCCTGAGGTGCTGGGCCCTGAGCTTCTACC     Base pairs
TGCGTATACTGAGTGGTGCGACAGAGACTGGTACTTCGGTGGGACTCCACGACCCGGGACTCGAAGATGG     631 to 700


CTGCGGAGATCACACTGACCTGGCAGCGGGATGGGGAGGACCAGACCCAGGACACGGAGCTCGTGGAGAC     Base pairs
GACGCCTCTAGTGTGACTGGACCGTCGCCCTACCCCTCCTGGTCTGGGTCCTGTGCCTCGAGCACCTCTG     701 to 770


CAGGCCTGCAGGGGATGGAACCTTCCAGAAGTGGGCGGCTGTGGTGGTGCCTTCTGGACAGGAGCAGAGA     Base pairs
GTCCGGACGTCCCCTACCTTGGAAGGTCTTCACCCGCCGACACCACCACGGAAGACCTGTCCTCGTCTCT     771 to 840


TACACCTGCCATGTGCAGCATGAGGGTTTGCCCAAGCCCCTCACCCTGAGATGGGAGCCGTCTTCCCAGC     Base pairs
ATGTGGACGGTACACGTCGTACTCCCAAACGGGTTCGGGGAGTGGGACTCTACCCTCGGCAGAAGGGTCG     841 to 910


CCACCATCCCCATCGTGGGCATCATTGCTGGCCTGGTTCTCTTTGGAGCTGTGATCACTGGAGCTGTGGT     Base pairs
GGTGGTAGGGGTAGCACCCGTAGTAACGACCGGACCAAGAGAAACCTCGACACTAGTGACCTCGACACCA     911 to 980


CGCTGCTGTGATGTGGAGGAGGAAGAGCTCAGATAGAAAAGGAGGGAGCTACTCTCAGGCTGCAAGCAGT     Base pairs
GCGACGACACTACACCTCCTCCTTCTCGAGTCTATCTTTTCCTCCCTCGATGAGAGTCCGACGTTCGTCA     981 to 1050


GACAGTGCCCAGGGCTCTGATGTGTCTCTCACAGCTTGTAAAGTGTGA     Base pairs
CTGTCACGGGTCCCGAGACTACACAGAGAGTGTCGAACATTTCACACT     1051 to 1098
```

A2-F1:       aaacccgggtctagaGGATGGCCGTCATGGCGCC

A2-R1:       cccgcggccgcTCACACTTTACAAGCTGTGAGAGAC

# Figure 12

```
ATGTCTCGCTCCGTGGCCTTAGCTGTGCTCGCGCTACTCTCTCTTTCTGGCCTGGAGGCTATCCAGCGTA    Base pairs
TACAGAGCGAGGCACCGGAATCGACACGAGCGCGATGAGAGAGAAAGACCGGACCTCCGATAGGTCGCAT    1 to 70


CTCCAAAGATTCAGGTTTACTCACGTCATCCAGCAGAGAATGGAAAGTCAAATTTCCTGAATTGCTATGT    Base pairs
GAGGTTTCTAAGTCCAAATGAGTGCAGTAGGTCGTCTCTTACCTTTCAGTTTAAAGGACTTAACGATACA    71 to 140


GTCTGGGTTTCATCCATCCGACATTGAAGTTGACTTACTGAAGAATGGAGAGAGAATTGAAAAAGTGGAG    Base pairs
CAGACCCAAAGTAGGTAGGCTGTAACTTCAACTGAATGACTTCTTACCTCTCTCTTAACTTTTTCACCTC    141 to 210


CATTCAGACTTGTCTTTCAGCAAGGACTGGTCTTTCTATCTCTTGTACTACACTGAATTCACCCCCACTG    Base pairs
GTAAGTCTGAACAGAAAGTCGTTCCTGACCAGAAAGATAGAGAACATGATGTGACTTAAGTGGGGGTGAC    211 to 280


AAAAAGATGAGTATGCCTGCCGTGTGAACCATGTGACTTTGTCACAGCCCAAGATAGTTAAGTGGGATCG    Base pairs
TTTTTCTACTCATACGGACGGCACACTTGGTACACTGAAACAGTGTCGGGTTCTATCAATTCACCCTAGC    281 to 350


AGACATGTAA    Base pairs
TCTGTACATT    351 to 360
```

Figure 13

Figure 14

**Figure 15**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BERNABEU et al.** *Nature,* 1984, vol. 308, 642-5
- **COOK et al.** *J Immunol,* 1996, vol. 157, 2256-61
- **HORIG et al.** *Proc Natl Acad Sci U S A,* 1997, vol. 94, 13826-31
- **HYAFIL ; STROMINGER.** *Proc Natl Acad Sci U S A,* 1979, vol. 76, 5834-8
- **LUSCHER et al.** *J Immunol,* 1994, vol. 153, 5068-81
- **PARKER et al.** *J Immunol,* 1992, vol. 149, 1896-904
- **SMITH et al.** *Proc Natl Acad Sci U S A,* 1992, vol. 89, 7767-71
- **WILLCOX et al.** *Immunity,* 1999, vol. 10, 357-65
- **WYER et al.** *Immunity,* 1999, vol. 10, 219-225
- **DING et al.** *Immunity,* 1999, vol. 11, 45-56
- **DING et al.** *Immunity,* 1998, vol. 8, 403-11
- **GARBOCZI et al.** *Nature,* 1996, vol. 384, 134-41
- **GARCIA et al.** *Science,* 1996, vol. 274, ISSN 0036-8075, 209-19
- **GAO et al.** *Nature,* 1997, vol. 387, 630-4
- **MESCHER.** *Immunol Rev,* 1995, vol. 146, 177-210
- **NORMENT.** *Nature,* 1988, vol. 336, 79-81
- **BERTOLETTI et al.** *Nature,* 1994, vol. 369, ISSN 0028-0836, 407-10
- **KLENERMAN et al.** *Nature,* 1994, vol. 369, 403-7
- **PURBHOO et al.** *Proc. Natl. Acad. Sci. USA,* 1998, vol. 95, 4527-4532
- **DE FAZIO et al.** *Transplantation,* 1996, vol. 61, 104-10
- **CLAYBERGER et al.** *J Immunol,* 1994, vol. 153, 946-51
- **CHOKSI et al.** *Nature Medicine,* 1998, vol. 4, 309-314
- **SEWELL et al.** *Nature Medicine,* 1999, vol. 5, 399-404
- **GAO et al.** *Prot. Sci.,* 1998, vol. 7, 1245-49
- **GARBOCZI et al.** *Proc Natl Acad Sci U S A,* 1992, vol. 89, ISSN 0027-8424, 3429-33
- **PARKER ; WILEY.** *Gene,* 1989, vol. 83, 117-24
- **FUKAZAWA et al.** *J Immunol,* 1994, vol. 153, 3543-50
- **IVANYI.** *Curr Opin Rheumatol,* 1992, vol. 4, 484-93
- **REVEILLE.** *Am J Med Sci,* 1998, vol. 316, 239-49
- **ALTMAN et al.** *Ann Allergy,* 1994, vol. 72, 307-16
- **VAN EYNDHOVEN et al.** *Clin Immunol Immunopathol,* 1994, vol. 72, 362-72
- **SCHULTZ et al.** *Immunogenetics,* 1998, vol. 48, 273-82
- **WALTER et al.** *J Immunol Methods,* 1998, vol. 214, 41-50
- **TRYMBULAK et al.** *Immunogenetics,* 1997, vol. 46, 418-426
- **J. SAMBROOK et al.** Molecular Cloning. Cold Spring Harbor Laboratory Press, 1989
- **SAMBROOK et al.** MOLECULAR CLONING, A LABORATORY MANUAL. Cold Spring Harbor Laboratory Press, 1989
- **FOECKING ; HOFSTETTER.** *Cell,* 1986, vol. 45, 101-105
- **DAVIS et al.** *BASIC METHODS IN MOLECULAR BIOLOGY,* 1986
- **SAMBROOK et al.** MOLECULAR CLONING: A LABORATORY MANUAL. Cold Spring Harbor Laboratory Press, 1989
- **GARCIA et al.** *Nature,* 1996, vol. 384, ISSN 0028-0836, 577-81
- **BJORKMAN et al.** *J Mol Biol,* 1985, vol. 186, 205-10
- **GARBOCZI et al.** *J Mol Biol,* 1994, vol. 239, ISSN 0022-2836, 581-7
- **MADDEN et al.** *Cell,* 28 January 1994, vol. 76 (2), 410
- *Cell,* 1993, vol. 75, ISSN 0092-8674, 693-708
- **REID et al.** *J Exp Med,* 1996, vol. 184, 2279-86
- **REID et al.** *FEBS Lett,* 1996, vol. 383, 119-23
- **SMITH et al.** *Immunity,* 1996, vol. 4, ISSN 1074-7613, 215-28
- **SMITH et al.** *Immunity,* 1996, vol. 4, ISSN 1074-7613, 203-13
- *Pharmaceutical Research,* 1986, vol. 3 (6), 318
- **STUDIER et al.** *Methods Enzymol,* 1990, vol. 185, ISSN 0076-6879, 60-89

- **SCHATZ.** *Biotechnology N Y,* 1993, vol. 11, 1138-43
- **ALTMAN et al.** *Science,* 1996, vol. 274, 94-6
- **O'CALLAGHAN et al.** *Anal Biochem,* 1999, vol. 266 (1), 9-15
- **PAYNE et al.** *Immunogenetics,* 1990, vol. 31 (3), 169-78
- **LEIST et al.** *J Immunol,* 1987, vol. 138, 2278-81